(19) Europäisches Patentamt
European Patent Office
Office européen des brevets

(11) EP 4 685 227 A1

(12) EUROPEAN PATENT APPLICATION
published in accordance with Art. 153(4) EPC

(43) Date of publication:
**28.01.2026 Bulletin 2026/05**

(21) Application number: **24775211.6**

(22) Date of filing: **21.03.2024**

(51) International Patent Classification (IPC):
***C12N 1/20*** *(2026.01)* ***A61K 8/99*** *(2017.01)*
***A61Q 19/00*** *(2006.01)*

(52) Cooperative Patent Classification (CPC):
**A61K 8/99; A61Q 19/00; C12N 1/20**

(86) International application number:
**PCT/KR2024/003544**

(87) International publication number:
**WO 2024/196165 (26.09.2024 Gazette 2024/39)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **23.03.2023 KR 20230038195**

(71) Applicant: **Cosmax, Inc.**
**Hwaseong-si, Gyeonggi-do 18622 (KR)**

(72) Inventors:
- **JO, Hyung Woo**
  **Seongnam-si, Gyeonggi-do 13361 (KR)**
- **KIM, Min Ji**
  **Suwon-si, Gyeonggi-do 16484 (KR)**
- **KIM, Mi Sun**
  **Suwon-si, Gyeonggi-do 16531 (KR)**
- **LEE, Dong Geol**
  **Hwaseong-si, Gyeonggi-do 18496 (KR)**
- **KANG, Seung Hyun**
  **Seoul 06285 (KR)**

(74) Representative: **Lavoix**
**Bayerstraße 83**
**80335 München (DE)**

# (54) MICROBIAL CULTURE MEDIUM COMPRISING HUMAN SKIN-DERIVED PRODUCTS, AND USE FOR SKIN IMPROVEMENT, OF MIXED MICROBIAL CULTURE SOLUTION CULTURED USING SAME

(57) The present invention relates to: a medium composition comprising sugars and amino acids derived from human skin; and a cosmetic composition comprising microorganisms of the genus *Staphylococcus,* the genus *Streptococcus,* the genus *Cutibacterium,* the genus *Corynebacterium* and the genus *Rothia,* or a lysate or culture solution thereof.

A mixed culture solution of human skin-derived microorganisms included in the cosmetic composition is cultured in the medium composition, exhibiting significantly excellent effects on skin improvement as compared with a culture solution of each microorganism.

EP 4 685 227 A1

# FIG. 1

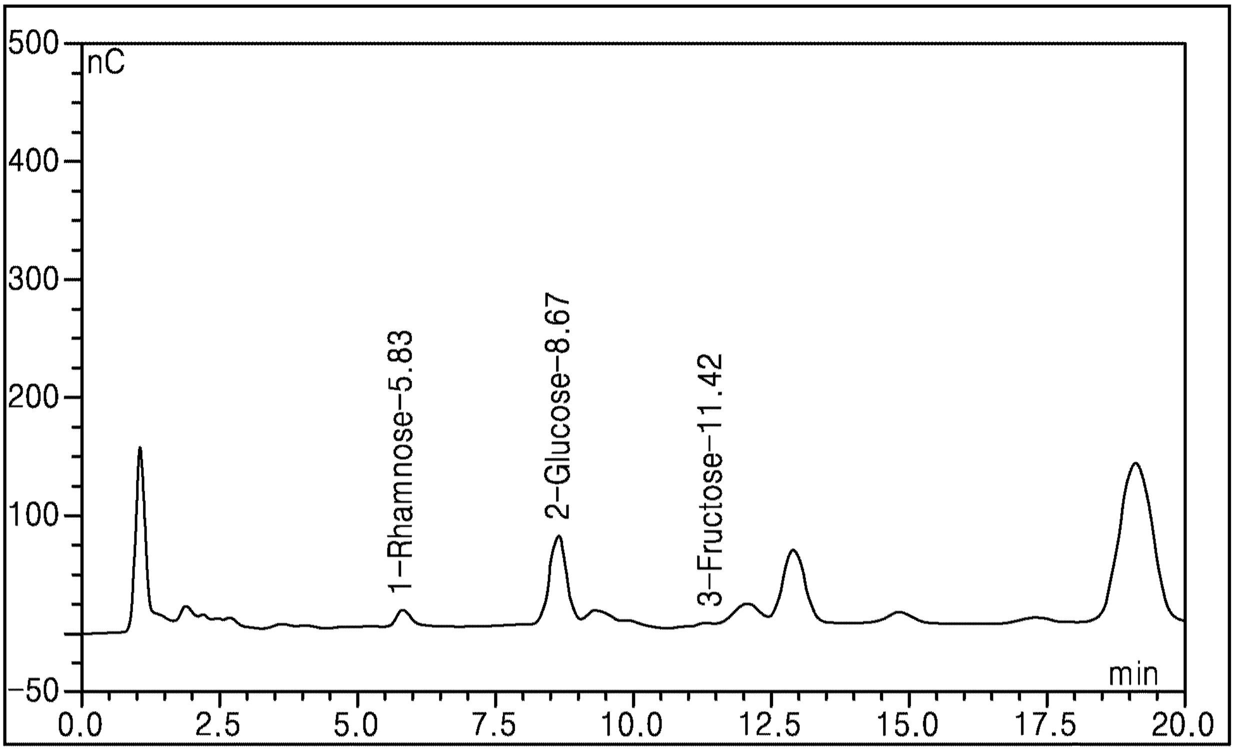


| No. | Ret.Time min | Peak Name | Height nC | Area nC*min | Rel.Area % | Amount (mg/mL) |
|---|---|---|---|---|---|---|
| 1 | 5.83 | Rhamnose | 14.596 | 4.249 | 13.24 | 0.003 |
| 2 | 8.67 | Glucose | 75.012 | 26.367 | 82.18 | 0.016 |
| 3 | 11.42 | Fluctose | 3.496 | 1.469 | 4.58 | 0.002 |
| Total : | | | 93.104 | 32.086 | 100.0 | 0.022 |

## Description

### Technical Field

The present disclosure relates to: a medium composition including human skin-derived sugars and amino acids; and a cosmetic composition including microorganisms of the genus *Staphylococcus,* the genus *Streptococcus,* the genus *Cutibacterium,* the genus *Corynebacterium* and the genus *Rothia,* or a lysate or culture solution thereof.

### Background Art

As general characteristics of skin, the skin surface is cold, slightly acidic, and remains dry. Structurally, the epidermis forms the skin barrier, blocking penetration of microorganisms and toxins, and plays an important role in maintaining moisture and body temperature through sweating. The outermost layer of the epidermis consists of the stratum corneum. The epidermis has a structure commonly referred to as 'brick and mortar structure,' in which skin tissue undergoes a continuous self-repair process, and squames that have undergone the final stage of differentiation are repeatedly shed from the skin tissue.

The skin barrier consists of dead skin cells and intercellular lipids, and acts as a protective layer that protects the skin from external stimuli and prevents moisture from evaporating from the skin, playing a key role in skin health. That is, it prevents excessive moisture loss from the body and blocks harmful substances such as chemicals and microorganisms from entering the body. In addition, the outer layer of keratinocytes, which constitutes the surface of dead keratinocytes, plays an important role in the stability of intercellular lipids. Keratinocytes form the skin barrier through differentiation and keratinization. The skin barrier function may become damaged through aging or external factors, and damage to the skin barrier can cause a decrease in skin moisture levels and the formation of wrinkles.

Meanwhile, during the processes above, sweat, keratin cells, lipid cells, and the like are produced in the skin, which also includes various substances such as sugar ingredients and amino acids. These substances can have a significant impact on the growth of microorganisms present on the skin and their role in skin improvement. Nevertheless, research on these substances and studies on microorganisms cultured using these substances and efficacy of these substances are still inadequate.

In response, the inventors of the present disclosure analyzed human skin-derived substances such as sugars and amino acids, to prepare a microbial culture medium including these substances, and analyzed useful microorganisms derived from human skin to mix and culture these microorganisms in the culture medium. Accordingly, it was found that the resulting culture has excellent skin-improving effects, thereby completing the present disclosure.

### Disclosure of Invention

### Technical Problem

One aspect is to provide a medium composition for promoting growth of human skin-derived microorganisms, the medium composition comprising human skin-derived sugars and amino acids.

Another aspect is to provide a method of preparing a cosmetic composition for skin improvement, the method including: inoculating and culturing each of microorganisms of the genus *Staphylococcus,* the genus *Streptococcus,* the genus *Cutibacterium,* the genus *Corynebacterium,* or the genus *Rothia,* in the medium composition to prepare a culture solution; centrifuging and filtering each culture solution to collect a supernatant of each culture solution; and mixing each supernatant.

Another aspect is to provide a cosmetic composition for skin improvement prepared by the method.

Another aspect is to provide use of: microorganisms of the genus *Staphylococcus,* the genus *Streptococcus,* the genus *Cutibacterium,* the genus *Corynebacterium,* or the genus *Rothia*; a culture solution obtained by culturing the microorganisms, a concentrate thereof, a lyophilisate thereof, a supernatant obtained by removing strains from the culture solution, and a mixture thereof; or the cosmetic composition.

### Solution to Problem

One aspect provides a medium composition for promoting growth of human skin-derived microorganisms, the medium composition comprising human skin-derived sugars and amino acids. In the present specification, the term 'medium composition' may be used interchangeably with 'novel medium,' 'modified medium,' and the like. Specifically, the sugars may be glucose, fructose, and rhamnose, and the amino acids may be threonine, tyrosine, arginine, alanine, proline, histidine, iso-leucine, leucine, phenyl-alanine, tryptophan, valine, glutamic acid, aspartic acid, serine and glycine. The sugars, amino acids, and microorganisms may be derived from human skin.

More specifically, the glucose may be included in an amount of 0.01 to 100 gram (g), 0.01 to 10 g, 0.01 to 5 g, 0.1 to 100 g, 0.1 to 10 g, 0.1 to 5 g, 1.0 to 100 g, 1.0 to 10 g, or 1.0 to 5 g, per 1 liter (L) of the medium composition, and the fructose and rhamnose may be included in an amount of 0.01 to 100 g, 0.01 to 10 g, 0.01 to 5 g, 0.1 to 100 g, 0.1 to 10 g, or 0.1 to 5 g, per 1 L of the medium composition. The threonine, tyrosine, arginine, alanine, proline, histidine, isoleucine, leucine, tryptophan, glutamic acid, aspartic acid, serine and glycine may be included in an amount of 0.1 milligram (mg) to 5.0 g, 0.1 mg to 1.0 g, 0.1 mg to 100 mg, or 0.1 mg to 50 mg, and the phenyl-alanine may be included in an amount of 0.1 mg to 5.0 g, 0.1 mg to 1.0 g, 0.1 mg to 100 mg, 0.1 mg to 50 mg, or 0.1 mg to 10 mg.

In an embodiment, samples were obtained by cleaning the facial area of subjects aged 10 to 20 with sterile distilled water, and sugars, amino acids, and microbiomes constituting the samples were analyzed. As a result, glucose, fructose, and rhamnose were detected as the sugar ingredients constituting the human skin-derived samples, and threonine, tyrosine, arginine, alanine, proline, histidine, iso-leucine, leucine, phenyl-alanine, tryptophan, valine, glutamic acid, aspartic acid, serine and glycine were detected as the amino acids constituting the human skin-derived samples.

The medium composition may be for promoting the growth of human skin-derived microorganisms, and specifically, the human skin-derived microorganisms may include any one or more selected from the group consisting of *Staphylococcus epidermidis, Streptococcus thermophilus, Cutibacterium acnes, Corynebacterium amycolatum,* and *Rothia kristinae.*

Specifically, the microorganism of the genus *Staphylococcus* may be *Staphylococcus epidermidis* KCCM12551P, the microorganism of the genus *Streptococcus* may be *Streptococcus thermophilus* KCCM12004P, the microorganism of the genus *Cutibacterium* may be *Cutibacterium acnes* KCCM12680P, the microorganism of the genus *Corynebacterium* may be *Corynebacterium amycolatum* KCCM12689P, and the microorganism of the genus *Rothia* may be *Rothia kristinae* KCCM12685P.

The medium composition may further include any one or more selected from the group consisting of glycerol, magnesium sulfate ($MgSO_4$), potassium phosphate ($K_2HPO_4$), sodium chloride (NaCl), and yeast extract, but ingredients are not limited thereto, provided that the ingredients included in the medium composition are capable of culturing the strains or promoting the growth of the strains.

Another aspect provides a method of preparing a cosmetic composition for skin improvement, the method including: inoculating and culturing each of microorganisms of the genus *Staphylococcus,* the genus *Streptococcus,* the genus *Cutibacterium,* the genus *Corynebacterium,* or the genus *Rothia,* in the medium composition to prepare a culture solution; centrifuging and filtering each culture solution to collect a supernatant of each culture solution; and mixing each supernatant. The same contents as described above also apply to the descriptions of the method.

Specifically, the microorganism of the genus *Staphylococcus* may be *Staphylococcus epidermidis* KCCM12551P, the microorganism of the genus *Streptococcus* may be *Streptococcus thermophilus* KCCM12004P, the microorganism of the genus *Cutibacterium* may be *Cutibacterium acnes* KCCM12680P, the microorganism of the genus *Corynebacterium* may be *Corynebacterium amycolatum* KCCM12689P, and the microorganism of the genus *Rothia* may be *Rothia kristinae* KCCM12685P.

In the preparation of the culture solution, the culturing temperature may preferably be from 25 °C to 37 °C, and the culturing time may be from 24 hours to 120 hours, and in the collection of the supernatant by the centrifuging and filtering, the centrifugation may be performed under conditions of 4,000 revolution per minute (rpm) to 8,000 rpm for 10 minutes to 30 minutes.

In an embodiment, in the preparation of the culture solution, the microorganisms are each inoculated into the medium composition and cultured at 30 °C for 72 hours to prepare the culture solution. Afterwards, each culture solution was centrifuged at 6,000 rpm for 30 minutes, and then, the resulting culture solution was filtered through a 0.45 micrometer ($\mu$m) filter to collect the supernatant. Also, to confirm the synergistic effect of a mixture of the supernatants of the culture solutions of each strain, the supernatants of the five types of microbial culture solutions were mixed at a ratio of 1:1:1:1:1 (v/v) to produce a mixture.

In the present specification, the term "culture solution" may refer to the entire medium, including strains, metabolites thereof, extra nutrients, and the like, obtained by culturing the microorganisms for a period of time in a medium capable of providing nutrients to enable the microorganisms to survive and grow in vitro, and such a culture solution may also include a culture solution itself obtained by culturing the microorganisms, or a concentrate or a lyophilisate of the culture solution.

In the present specification, the term "supernatant" may refer to a liquid from which the culture has been cleared of strains, and may be obtained by allowing the culture solution to stand still for a period of time and taking only the liquid from the upper layer, excluding the portion that has settled to the bottom layer, by removing strains by filtration, or by centrifuging the culture solution to remove precipitates and strains and taking only the liquid from the upper layer.

Regarding the method of preparing the cosmetic composition for skin improvement, the skin improvement may include skin moisturization, skin barrier strengthening, skin whitening, wrinkle reduction, skin texture improvement, pore reduction, skin tone improvement, skin elasticity improvement, or skin density improvement, but is not particularly limited thereto.

In the present specification term "skin moisturization" refers to any action that maintains moisture in the skin or prevent loss of moisture, and the term "skin barrier strengthening" refers to any action that promotes the function of the skin barrier located on the outermost layer of the skin to prevent loss of moisture and the like. The term "skin whitening" refers to brightening the skin tone by inhibiting synthesis of melanin pigment in the skin as well as ameliorating hyperpigmentation such as freckles and age spots caused by ultraviolet rays, hormones, or genetic reasons, and the term "wrinkle reduction" refers to any action that resolves the condition of skin that loosens or folds due to loss of elasticity. The term "skin texture improvement" refers to any action that reduces the gathering of hard or soft parts of skin tissue and the occurrence of unevenness, and the term "pore reduction" refers to any action that reduces the area and number of pores visible to the naked eye on the skin. The term "skin tone improvement" may refer to any action that makes the skin color whiter and brighter by increasing the individual typology angle (ITA) value, and the term "skin elasticity improvement or skin density improvement" may refer to any action that restores skin elasticity by increasing the ability of collagen, which is a connective fibre in the dermis, and mucopolysaccharides, which are mucous substances, to bind with water.

Another aspect provides a cosmetic composition for skin improvement prepared by the method. The same contents as described above also apply to the descriptions of the cosmetic composition.

Specifically, the cosmetic composition may include microorganisms of the genus *Staphylococcus,* the genus *Streptococcus,* the genus *Cutibacterium,* the genus *Corynebacterium* and the genus *Rothia,* a culture solution of the microorganisms, or a supernatant of the culture solution. The microorganism of the genus the *Staphylococcus* may be *Staphylococcus epidermidis* KCCM12551P, the microorganism of the genus *Streptococcus* may be *Streptococcus thermophilus* KCCM12004P, the microorganism of the genus *Cutibacterium* may be *Cutibacterium acnes* KCCM12680P, the microorganism of the genus *Corynebacterium* may be *Corynebacterium amycolatum* KCCM12689P, and the microorganism of the genus *Rothia* may be *Rothia kristinae* KCCM12685P. Also, the cosmetic composition may include the microbial culture solution prepared by culturing in the medium composition including the human skin-derived sugars and amino acids.

Specifically, the cosmetic composition may include, as an active ingredient, the microorganisms, a lysate thereof, a culture solution obtained by culturing the microorganisms, a concentrate, extract, and lyophilisate of the culture solution, a supernatant obtained by removing the microorganisms from the culture solution, and a mixture thereof.

The skin improvement may include skin moisturization, skin barrier strengthening, skin whitening, wrinkle reduction, skin texture improvement, pore reduction, skin tone improvement, skin elasticity improvement, or skin density improvement, but is not specifically limited thereto.

In the cosmetic composition, the microorganisms of the genus *Staphylococcus,* the genus *Streptococcus,* the genus *Cutibacterium,* the genus *Corynebacterium* and the genus *Rothia,* culture solutions of the microorganisms, or supernatants of the culture solutions may be mixed at a ratio of 1 to 10:1 to 10:1 to 10:1 to 10:1 to 10 (v/v), specifically at the same volume ratio, i.e., 1:1:1:1:1 (v/v).

The cosmetic composition may further include in addition to the ingredients described in the present specification, functional additives and ingredients included in general cosmetic compositions, and conventional supplementary ingredients, such as antioxidants, stabilizers, solubilizers, vitamins, pigments, and flavoring agents, etc., and carriers may be further included. For example, the cosmetic composition may additionally include supplementary ingredients such as glycerin, butylene glycol, polyoxyethylene hydrogenated castor oil, tocopherol acetate, citric acid, squalane, sodium citrate, allantoin, etc., and may include hexanediol or distilled water as a solvent. Also, the functional additive may include ingredients selected from the group consisting of water-soluble vitamins, fat-soluble vitamins, polymer peptides, polymer polysaccharide, sphingolipids, and seaweed extract. Other mixing ingredients included in the cosmetic composition may include oil and fat ingredients, moisturizers, emollients, surfactants, organic and inorganic pigments, organic powders, ultraviolet (UV) absorbers, preservatives, antiseptics, antioxidants, plant extracts, pH adjusters, alcohol, colorants, flavoring agents, blood circulation promoters, cooling agents, restricted ingredients, distilled water, and the like, but are not particularly limited thereto.

Another aspect provides use of: microorganisms of the genus *Staphylococcus,* the genus *Streptococcus,* the genus *Cutibacterium,* the genus *Corynebacterium,* or the genus *Rothia*; a culture solution obtained by culturing the microorganisms, a concentrate thereof, a lyophilisate thereof, a supernatant obtained by removing strains from the culture solution, and a mixture thereof; or the cosmetic composition.

Specifically, the microorganism of the genus *Staphylococcus* may be *Staphylococcus epidermidis* KCCM12551P, the microorganism of the genus *Streptococcus* may be *Streptococcus thermophilus* KCCM12004P, the microorganism of the genus *Cutibacterium* may be *Cutibacterium acnes* KCCM12680P, the microorganism of the genus *Corynebacterium* may be *Corynebacterium amycolatum* KCCM12689P, and the microorganism of the genus *Rothia* may be *Rothia kristinae* KCCM12685P.

The use may include skin condition amelioration, skin moisturization, skin barrier strengthening, skin whitening, wrinkle reduction, skin texture improvement, pore reduction, skin tone improvement, skin elasticity improvement, skin density improvement, or prevention, alleviation, or treatment of skin diseases.

Also, the use may include use for preparing: a composition for skin improvement; a composition for skin moisturization, skin barrier strengthening, skin whitening, wrinkle reduction, skin texture improvement, pore reduction, skin tone improvement, skin elasticity improvement, or skin density improvement; or a composition for prevention, alleviation, or treatment of skin diseases.

## Advantageous Effects of Invention

A mixed culture solution of human skin-derived microorganisms included in the cosmetic composition of the present disclosure is cultured in the medium composition, exhibiting significantly excellent effects on skin improvement as compared with a culture solution of each microorganism.

## Brief Description of Drawings

FIG. 1 shows results of analyzing sugar ingredients of skin-derived products obtained by cleaning the facial skin of subjects, according to an embodiment.

FIG. 2 shows results of analyzing amino acid ingredients of skin-derived products obtained by cleaning the facial skin of subjects, according to an embodiment.

FIG. 3 shows the distribution of skin-derived microorganisms in skin-derived products obtained by cleaning the facial skin of subjects, according to an embodiment.

FIGS. 4 and 5 show results of evaluating moisturizing effects of skin-derived microorganisms cultured in a newly developed, modified medium.

FIG. 6 shows results of evaluating barrier improvement effects of skin-derived microorganisms cultured in a newly developed, modified medium.

FIGS. 7 and 8 show results of comparing moisturizing effects of skin-derived microorganisms, which are cultured in a newly developed, modified medium, and mixtures thereof.

FIG. 9 shows results of comparing barrier improvement effects of skin-derived microorganisms, which are cultured in a newly developed, modified medium, and mixtures thereof.

FIG. 10 shows results of evaluating whitening effects of skin-derived microorganisms cultured in a newly developed, modified medium.

FIGS. 11 and 12 show results of evaluating anti-aging and wrinkle-reducing effects of skin-derived microorganisms cultured in a newly developed, modified medium.

FIGS. 13 and 14 show results of comparing anti-aging and wrinkle-reducing effects of skin-derived microorganisms, which are cultured in a newly developed, modified medium, and mixtures thereof.

FIGS. 15 and 16 show results of a clinical evaluation of the reduction of wrinkles around the eyes by using a formulation containing 5 types of microbial culture solutions (Ra: arithmetic mean roughness, Rz: 10-point mean roughness).

FIGS. 17 and 18 show results of a clinical evaluation of skin texture improvement using a formulation containing 5 types of microbial culture solutions (Ra: average roughness, Rmax: maximum roughness, Rz: 10-point average roughness; difference between an average length value of five highest peaks and an average length value of five lowest valleys from the average line, Rp: maximum cross-sectional peak height; a height value of the highest cross-sectional peak from the center line of the cross-sectional roughness, Rv: maximum cross-sectional valley height, a depth value of the lowest cross-sectional valley from the center line of the cross-sectional roughness).

FIGS. 19 and 20 show results of a clinical evaluation of skin moisture improvement using a formulation containing 5 types of microbial culture solutions.

FIGS. 21 and 22 show results of a clinical evaluation of skin pore reduction using a formulation containing 5 types of microbial culture solutions.

FIGS. 23 and 24 show results of a clinical evaluation of skin tone improvement using a formulation containing 5 types of microbial culture solutions.

FIG. 25 shows results of a clinical evaluation of skin elasticity improvement using a formulation containing 5 types of microbial culture solutions (R2 (Ua/Uf): ability (%) to return to normal after resistance to physical force, R5 (Ua/Ue): ratio (%) of suction elasticity to relaxation elasticity, R7 (Ur/Uf): ratio (%) of elasticity to total curve).

FIGS. 26 and 27 show results of a clinical evaluation of skin density improvement using a formulation containing 5 types of microbial culture solutions.

## Best Mode for Carrying out the Invention

## Mode for the Invention

Hereinafter, the present disclosure will be described in more detail with reference to Examples below. However, these Examples are for illustrative purposes only, and the scope of the present disclosure is not intended to be limited by these Examples.

### Example 1. Skin-derived products and microbiome sampling

Participants aged 10 to 20 years old were recruited. After cleansing their faces, the participants rested for at least 30 minutes in a constant temperature and humidity chamber (22±2 °C, 50±5 %). Then, samples were obtained by cleansing their faces with sterile distilled water. These samples were used for analyzing constituent sugars, amino acids, and microbiome.

### Example 2. Analysis of constituent sugars in skin-derived product

The samples obtained in Example 1 were powdered through a freeze-drying process. The powdered samples were then analyzed for constituent sugars using BIO-LC equipment, and the results are shown in FIG. 1. As a result, it was confirmed that glucose, fructose, and rhamnose were detected on the skin as sugars available as energy sources for microorganisms.

### Example 3. Analysis of amino acids in skin-derived product

The samples obtained in Example 1 were powdered through a freeze-drying process. The powdered samples were then analyzed for free amino acids using an amino acid analyzer, and the results are shown in FIG. 2. As a result, it was confirmed that threonine, tyrosine, arginine, alanine, proline, histidine, isoleucine, leucine, phenyl-alanine, tryptophan, valine, glutamic acid, aspartic acid, serine and glycine were detected on the skin as nitrogen sources available as energy sources for microorganisms.

### Example 4. Analysis of skin microorganisms through next generation sequencing (NGS)

The samples obtained in Example 1 were filtered through a 0.45 μm filter, and only the filter membrane was collected to extract metagenomic DNA. The extracted DNA was amplified in the V3-V4 region of the 16S rRNA gene region for NGS analysis, and an amplicon-type DNA library was produced. Impurities other than DNS were then removed through a purification process. The produced DNA library was analyzed using Miseq equipment (Illumina, Inc.), and the obtained data were analyzed using the Qiime2 application and shown in FIG .3.

As a result, it was confirmed through the analysis that microorganisms of the genus *Staphylococcus,* the genus *Streptococcus,* the genus *Cutibacterium,* the genus *Corynebacterium* and the genus *Rothia* are strains present in the skin. Specifically, it was confirmed that the microorganism of the genus *Staphylococcus* was *Staphylococcus epidermidis,* the microorganism of the genus *Streptococcus* was *Streptococcus thermophilus,* the microorganism of the genus *Cutibacterium* was *Cutibacterium acnes,* and the microorganism of the genus *Rothia* was *Rothia kristinae.*

### Example 5. Preparation of new microbial culture medium

Based on the skin-derived sugars and amino acids identified in Examples 2 and 3, a new microbial culture medium containing ingredients at amounts as listed in Table 1 was prepared.

Table 1

| **Ingredient** | Amount range |
|---|---|
| Glucose | 0.01 g - 100 g |
| Rhamnose | 0.01 g - 100 g |
| Fructose | 0.01 g - 100 g |
| Glycerol | 0.01 g - 50 g |
| Threonine | 0.1 mg - 5.0 g |
| Tyrosine | 0.1 mg - 5.0 g |
| Arginine | 0.1 mg - 5.0 g |

(continued)

| **Ingredient** | Amount range |
|---|---|
| Alanine | 0.1 mg - 5.0 g |
| Proline | 0.1 mg - 5.0 g |
| Histidine | 0.1 mg - 5.0 g |
| Iso-leucine | 0.1 mg - 5.0 g |
| Leucine | 0.1 mg - 5.0 g |
| Phenyl alanine | 0.1 mg - 5.0 g |
| Tryptophan | 0.1 mg - 5.0 g |
| Valine | 0.1 mg - 5.0 g |
| Glutamic acid | 0.1 mg - 5.0 g |
| Aspartate | 0.1 mg - 5.0 g |
| Serine | 0.1 mg - 5.0 g |
| Glycine | 0.1 mg - 5.0 g |
| $MgSO_4$ | 0.01 g - 1.0 g |
| $K_2HPO_4$ | 0.01 g - 5.0 g |
| NaCl | 0.01 g - 10.0 g |
| Yeast extract | 0.01 g - 10 g |

**Example 6. Preparation of culture solution of strains using medium containing skin-derived ingredients**

Based on the ingredients listed in Table 1, each strain belonging to the strain identified in Example 2, i.e., *Staphylococcus epidermidis, Streptococcus thermophilus, Cutibacterium acnes, Corynebacterium amycolatum,* and *Rothia kristinae,* was inoculated into a liquid medium and cultured at 30 °C for 72 hours to prepare a culture solution. Next, to collect only the supernatant, the culture solution was centrifuged at 6,000 rpm for 30 minutes, and then filtered through a 0.45 μm filter to collect only the completely sterilized supernatant.

**Example 7. Preparation of culture solution of strains using commercially available medium**

*Staphylococcus epidermidis, Corynebacterium amycolatum,* and *Rothia kristinae* strains were each inoculated into R2A medium, whereas *Streptococcus thermophilus* and *Cutibacterium acnes* strains were each inoculated into TSB medium. Then, the strains were cultured at 30 °C for 72 hours to prepare a culture solution. Next, to collect only the culture supernatant, the culture solution was centrifuged at 6,000 rpm for 30 minutes, and then filtered through a 0.45 μm filter to collect only the completely sterilized supernatant.

**Example 8. Preparation of mixture of supernatants of culture solution of strains using medium containing skin-derived ingredients**

Based on the ingredients listed in Table 1, each strain belonging to the strain identified in Example 2, i.e., *Staphylococcus epidermidis, Streptococcus thermophilus, Cutibacterium acnes, Corynebacterium amycolatum,* and *Rothia kristinae,* was inoculated into a liquid medium and cultured at 30 °C for 72 hours to prepare a culture solution. To collect only the culture supernatant, the culture solution was centrifuged at 6,000 rpm for 30 minutes, and then filtered through a 0.45 μm filter to collect only the completely sterilized supernatant. In addition, to confirm the synergistic effect of a mixture of the supernatants of the respective strains, the five supernatants were mixed at a ratio of 1:1:1:1:1 to prepare a mixture.

**Example 9. Comparison and analysis of skin moisturization/barrier strengthening effects**

The effects of the culture solutions or mixed culture solutions prepared in Examples 6 to 8 on skin barrier strengthening and skin moisturization were analyzed.

Specifically, HaCaT cells as a human keratinocyte cell line were cultured in Dulbecco's modified Eagle's medium (DMEM) (Gibco 1210-0038) supplemented with 10 % fetal bovine serum, and cultured in a 5 % $CO_2$ incubator at 37 °C. The cultured cell line was treated with the prepared culture solution (1 % (w/w)) of strains and mixture of the culture solutions (1 % (w/w)), and cultured for an additional 24 hours.

Next, RNA was isolated from the cells of each sample using Trizol (RNA iso, DAKARA, Japan), and quantified at 260 nanometer (nm) using a nanodrop. Then, cDNA was synthesized in an amplifier (C1000 Thermal Cycler, Bio-Rad, USA) using 2 μg of RNA of each sample. The synthesized cDNA was used as a template, and primers and cDNA that target HAS3, AQP3, and filaggrin (FLG) target genes as factors associated with skin barrier strengthening and skin moisturization were added together to perform real-time polymerase chain reaction (PCR) using a real-time PCR device (Step One Plus, Applied Biosystems, USA). Expression levels of the genes were finally analyzed by correction for the β-actin gene. Here, retinoic acid (RA) was used as a positive control group, and the results are shown in FIGS. 4 to 6.

As a result, it was confirmed that, among the strains, *Streptococcus thermophilus, Staphylococcus epidermidis, Rothia kristinae, Corynebacterium amycolatum,* and *Cutibacterium acnes* helped with skin moisturization by increasing the expression of HAS3 and AQP3 (see FIGS. 4 and 5), and *Staphylococcus epidermidis, Rothia kristinae, Corynebacterium amycolatum,* and *Cutibacterium acnes* helped with skin barrier strengthening by increasing the expression of FLG (see FIG. 6). In addition, the skin moisturizing/barrier strengthening efficacy of each strain was found to be generally higher when cultured in the new medium (modified medium) than in the commercially available medium.

In addition, as shown in FIGS. 7 to 9, it was confirmed that a mixture (Experimental example) of the supernatants of *Streptococcus thermophilus, Staphylococcus epidermidis, Rothia kristinae, Corynebacterium amycolatum,* and *Cutibacterium acnes* mixed at a ratio of 1:1:1:1:1 was more effective in skin barrier strengthening and skin moisturization than individual fermentation supernatants of the respective strains.

**Example 10. Analysis of whitening efficacy**

The effects of the culture solutions or mixed culture solutions prepared in Examples 6 to 8 on skin whitening were analyzed.

Specifically, B16-F10 (Murine Melanoma) cells (ATCC), which are pigment cells from mice, were suspended in 2 ml of DMEM medium (Gibco 1210-0038) supplemented with 10 % FBS, and seeded at a density of $1x10^5$ cells/well into a 6-well plate. The cells were then cultured in a 5 % $CO_2$ incubator at 37 °C until the cells reached 40 to 50 % confluence on the well bottom. Next, the cells were treated with α-MSH at a concentration of 100 nM to induce melanin production. Next, the cells were treated with the culture solution of the strain (1 % (w/w)) and cultured for an additional three days. Next, the culture solution was collected and centrifuged at 1,000 rpm for 10 minutes to obtain the supernatant. The obtained supernatant was subjected to absorbance measurement three times at 490 nm using a microplate reader (Victor 3) to evaluate effects on inhibition of melanin secretion . The following equation was used for the evaluation, and the results are shown in FIG. 7. Here, albutin (100 ppm, Sigma aldrich) was used as a positive control group.

Melanin contents (%) = (average absorbance of each sample-treated group)/(average absorbance of α-MSH-treated group) × 100 (%) [Equation 1]

As a result, it was confirmed that the *Corynebacterium amycolatum* strain significantly reduced melanin levels that have been increased by α-MSH as compared with the untreated control group, and when cultured in the new medium (modified medium), its melanin-reducing effect was found to be high as compared with the commercially available medium (see FIG. 10). Therefore, the strain may be useful in skin whitening.

**Example 11. Comparison and analysis of anti-aging activity**

The skin aging and wrinkle reduction activities were confirmed in the culture solutions of strains prepared in Examples 6 to 8.

Specifically, a human dermal fibroblast (Hs68) cell line was seeded at a density of $3.5x10^5$ cells into a 6-well plate, and then cultured for 24 hours in a 5% $CO_2$ incubator at 37 °C. Next, the medium was removed, and DPBS was added, and 20 mJ/$cm^2$ of ultraviolet B (UVB) was either irradiated or not irradiated. Immediately after UVB irradiation, the DPBS was removed, the medium was replaced with an FBS-free medium, and the cells were treated with 1 % culture solution of strain prepared in Examples 6 to 8 and then cultured for 24 hours. Here, a UV-treated group and a group not treated with the culture solution of strain were used as negative control groups, and epigallocatechin gallate (EGCG) was used as a positive control group. Next, RNA was isolated from the cells of each sample using Trizol (RNA iso, DAKARA, Japan), and quantified at 260 nanometer (nm) using a nanodrop. Then, cDNA was synthesized in an amplifier (C1000 Thermal Cycler, Bio-Rad, USA) using 2 μg of RNA of each sample. The synthesized cDNA was used as a template, and SYBR Green supermix (Applied Biosystems, USA) was added together with primers and cDNA that target COL1A1 and MMP1 target genes to perform a PCR using a real-time PCR device (Step One Plus, Applied Biosystems, USA). The expression levels of the genes were finally analyzed through correction for the β-actin gene.

As a result, it was confirmed that, among the strains, the *Streptococcus thermophilus,* the *Staphylococcus epidermidis,* the *Rothia kristina,* and the *Corynebacterium amycolatum* increased COL1A1 expression, and the *Corynebacterium amycolatum* decreased MMP-1 expression, thereby helping with anti-aging and wrinkle reduction (see FIGS. 11 and 12). Therefore, the strains were useful in skin anti-aging and wrinkle reduction.

Meanwhile, as shown in FIGS. 13 and 14, it was confirmed that the mixture of the supernatants of *Streptococcus thermophilus, Staphylococcus epidermidis, Rothia kristinae, Corynebacterium amycolatum,* and *Cutibacterium acnes* mixed at a ratio of 1:1:1:1:1 showed a greater increase in COL1A1 expression and a significant decrease in MMP-1 expression induced by UVB irradiation, as compared to individual fermentation supernatants of the respective strains, thereby demonstrating greater efficacy in anti-aging and wrinkle reduction.

**Example 12. Preparation of formulation containing strain culture solution**

Formulations for clinical evaluation were prepared using the 5 types of microbial culture solutions prepared in Example 6, and compositions and amounts of the formulations are as shown in Table 2.

Table 2

| **Ingredient** | **Amount (%)** |
|---|---|
| *Culture solution of Streptococcus thermophilus* | 5 |
| Culture solution of *Staphylococcus epidermidis* | 5 |
| Culture solution of *Corynebacterium amycolatum* | 5 |
| Culture solution of *Rothia kristinae* | 5 |
| Culture solution of *Cutibacterium acnes* | 5 |
| Sterile distilled water | 50.79 |
| 1,2-Hexandiol | 2 |
| Tromethamine | 0.16 |
| SC50 | 0.05 |
| Carbopol 980 | 20 |

**Example 13. Recruitment of subjects and experimental methods for formulation evaluation**

A total of 23 subjects aged 40 to 55 (average age of 48.78±2.24) who met the selection criteria were recruited through the H&BIO Skin Clinical Research Centre. The formulation prepared in Example 12 i.e., the test product, was applied to the designated facial areas of the subjects twice a day (morning and evening) for 4 weeks. Wrinkles around the eyes, skin pores, elasticity, and density were measured before use of the product and 4 weeks after use. Also, skin texture, moisture level, and skin tone were measured before use of the product, after one use of the product, after 3 days of use of the product, and after 4 weeks of use of the product. All studies were conducted after the subjects cleansed the test area and rested for at least 30 minutes in a constant temperature and humidity chamber (22±2 °C, 50±5 %).

**Example 14. Evaluation of efficacy of formulation in reducing wrinkles around eyes and improving skin texture (roughness)**

To evaluate efficacy of the formulation prepared in Example 12 in reducing wrinkles around the eyes and improving skin texture, the selected eye area was photographed before use of the product and after 4 weeks of use, and 3D images of the cheek area were taken before use of the product, after one use of the product, after 3 days of use of the product, and after 4 weeks of use of the product, and parameter values of the wrinkles around the eyes and skin texture and analyzed using an analysis program to analyze. The wrinkles around the eyes and skin texture were measured using a three-dimensional imaging system PRIMOSCR using a high-resolution sensor, and the results are shown in FIGS. 15 to 18.

As a result, it was confirmed that, as shown in FIGS. 15 and 16, the formulation was effective in wrinkle reduction by decreasing the average depth, size, number, length, and roughness (Rz) of wrinkles around the eyes, and that, as shown in FIGS. 17 and 18, the formulation was effective in skin texture improvement by reducing the average/maximum roughness parameters of the skin.

**Example 15. Efficacy of increasing skin moisture levels**

To evaluate efficacy of the formulation prepared in Example 12 in increasing skin moisture level, the moisture level of the selected cheek area was measured three times using a Corneometer CM 825 before use of the product, after one use of the product, after 3 days of use of the product, and after 4 weeks of use of the product, and the average value was analyzed. The selected forearm areas were photographed using Epsion Model E100, and the results are shown in FIGS. 19 and 20.

As a result, it was confirmed that, as shown in FIGS. 19 and 20, the formulation increased skin moisture levels compared to before use.

**Example 16. Efficacy of skin pore amelioration (reduction)**

To evaluate efficacy of the formulation prepared in Example 12 in skin pore amelioration (reduction), optical images photographed before use of the product and after 4 weeks of use were analyzed using Image-Pro 10 to measure the percentage (%) of pore spot areas, and the results are shown in FIGS. 21 and 22.

As a results, it was confirmed, as shown in FIGS. 21 and 22, the formulation reduced the area and number of skin pores compared to before use, thereby demonstrating effects in pore reduction.

**Example 17. Efficacy of improving skin tone**

To evaluate efficacy of the formulation prepared in Example 12 in skin tone improvement, the skin tone (Individual Typology Angle, ITA) of the selected cheek area was measured before use of the product, after one use of the product, after 3 days of use of the product, and after 4 weeks of use of the product, three times according to the following equation to analyze the average value, and the results are shown in FIGS. 23 and 24.

**[Equation 2]**

$$\text{ITA} = [\text{Arc Tangent } \{L^*-50\}/b^*]]\ 180 / 3.14159$$

Specifically, the optical system of spectrophotometer CM26dG that can measure skin tone measured the spectral reflectance and tristimulus value of samples using a method of diffuse illumination with an 8° angle equipped with an integrating sphere, and followed the CIE L*a*b* color coordinate system. In the L*a*b* color coordinate system, brightness is indicated by L*(lightness), and a* and b* indicate color and chroma in the direction of color. (a*: red, -a*: green, b*: yellow, -b*: blue direction).

As a result, it was confirmed that, as shown in FIGS. 23 and 24, the formulation has an effect of improving skin tone compared to before use.

**Example 18. Efficacy of improving skin elasticity**

To evaluate efficacy of the formulation prepared in Example 12 in improving skin tone, skin elasticity of the selected cheek area was measured before use of the product and 4 weeks after use of the product using Cutometer MPA580, and the R2, R5, and R7 parameters were analyzed and the results are shown in FIG. 25.

As a result, it was confirmed that, as shown in FIG. 25 and compared to before use, the formulation increased the skin recovery ability, the ratio of relaxation elasticity to suction elasticity, and the ratio of elasticity to the total curve, thereby demonstrating effects in skin elasticity improvement.

**Example 19. Efficacy of improving skin density**

To evaluate efficacy of the formulation prepared in Example 12 in improving skin tone, the skin density in the selected cheek area was measured before use of the product and after 4 weeks of the product using a DermaLab Series SkinLab Combo device. A measuring device is an ultrasonic imaging device that projects acoustic pulses onto the skin and measures the response rate. Based on the signal intensity, low density is output into dark color and high density is output into light color, and the results are shown in FIGS. 26 and 27.

As a result, it was confirmed that, as shown in FIGS. 26 and 27, the formulation has an effect of increasing skin density compared to before use.

The foregoing descriptions are only for illustrating the disclosure, and it will be apparent to a person having ordinary skill in the art to which the present invention pertains that the embodiments disclosed herein can be easily modified into other specific forms without changing the technical spirit or essential features. Therefore, it should be understood that Examples described herein are illustrative in all respects and are not limited.

## [Accession No.]

Name of Depository Authority: Korean Culture Center of Microorganisms (KCCM)

Accession No. : KCCM12551P

Accession date : 20190611

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. COSMAX CO., LTD.
46, Jeyakgongdan 2-gil,
Hyangnam-eup, Hwaseong-si,
Gyeonggi-do, 18622,
Republic of Korea

RECEIPT IN THE CASE OF AN ORIGINAL DEPOSIT
issued pursuant to Rule 7.1 by the
INTERNATIONAL DEPOSITARY AUTHORITY
identified at the bottom of this page

| I. IDENTIFICATION OF THE MICROORGANISM | |
|---|---|
| Identification reference given by the DEPOSITOR: *Staphylococcus epidermidis* ST-6 | Accession number given by the INTERNATIONAL DEPOSITARY AUTHORITY: KCCM12551P |
| II. SCIENTIFIC DESCRIPTION AND/OR PROPOSED TAXONOMIC DESIGNATION | |
| The microorganism identified under I above was accompanied by:<br>☐ a scientific description<br>☐ a proposed taxonomic designation<br>(Mark with a cross where applicable) | |
| III. RECEIPT AND ACCEPTANCE | |
| This International Depositary Authority accepts the microorganism identified under I above, which was received by it on June. 11. 2019 (date of the original deposit).[1] | |
| IV. RECEIPT OF REQUEST FOR CONVERSION | |
| The microorganism identified under I above was received by this International Depositary Authority on (date of the original deposit) and a request to convert the original deposit to a deposit under the Budapest Treaty was received by it on (date of receipt of request for conversion). | |
| V. INTERNATIONAL DEPOSITARY AUTHORITY | |
| Name : Korean Culture Center of Microorganisms<br>Address : Yurim B/D<br>45, Hongjenae-2ga-gil<br>Seodaemun-gu<br>SEOUL 03641<br>Republic of Korea | Signature(s) of person(s) having the power to represent the International Depositary Authority or of authorized official(s):<br>Date: June. 11. 2019 |

[1] Where Rule 6.4(d) applies, such date is the date on which the status of international depositary authority was acquired.

Form BP/4 (sole page)

한국미생물보존센터
03641 서울시 서대문구 홍제내2가길 45 유림빌딩 Tel: 02-391-0950, 396-0950 Fax: 02-392-2859
KOREAN CULTURE CENTER OF MICROORGANISMS
Yoolim Bldg., 45, Hongjenae 2ga-gil, Seodaemun-gu, Seoul, 03641, Korea Tel: 82-2-391-0950, 396-0950 Fax: 82-2-392-2859

Name of Depository Authority: Korean Culture Center of Microorganisms (KCCM)

Accession No. : KCCM12004P

Accession date : 20170403

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. COSMAX
9F, Pangyo-inno valley E,
255, Pangyo-ro, Bundang-gu,
Seongnam-si, Gyeonggi-do, 13486,
South Korea

RECEIPT IN THE CASE OF AN ORIGINAL
issued pursuant to Rule 7.1 by the
INTERNATIONAL DEPOSITARY AUTHORITY
identified at the bottom of this page

| I. IDENTIFICATION OF THE MICROORGANISM | |
|---|---|
| Identification reference given by the DEPOSITOR :<br>*Streptococcus* sp. CX-1 | Accession number given by the INTERNATIONAL DEPOSITARY AUTHORITY:<br>KCCM12004P |
| II. SCIENTIFIC DESCRIPTION AND/OR PROPOSED TAXONOMIC DESIGNATION | |
| The microorganism identified under I above was accompanied by:<br>☐ a scientific description<br>☐ a proposed taxonomic designation<br>(Mark with a cross where applicable) | |
| III. RECEIPT AND ACCEPTANCE | |
| This International Depositary Authority accepts the microorganism identified under I above, which was received by it on April. 03. 2017. (date of the original deposit)[1] | |
| IV. INTERNATIONAL DEPOSITARY AUTHORITY | |
| Name : Korean Culture Center of Microorganisms<br><br>Address : Yurim B/D<br>45, Hongjenae-2ga-gil<br>Seodaemun-gu<br>SEOUL 120-861<br>Republic of Korea | Signature(s) of person(s) having the power to represent the International Depositary Authority or of authorized official(s):<br><br>Date: April. 03. 2017. |

[1] Where Rule 6.4(d) applies, such date is the date on which the status of international depositary authority was acquired; where a deposit made outside the Budapest Treaty after the acquisition of the status of international depositary authority is converted into a deposit under the Budapest Treaty, such date is the date on which the microorganism was received by the international depositary authority.

Form BP/4 Sole page

한국미생물보존센터
03641 서울시 서대문구 홍제내2가길 45 유림빌딩 Tel 02-391-0950, 396-0950 Fax: 02-392-2859
KOREAN CULTURE CENTER OF MICROORGANISMS
Yoolim Bldg., 45, Hongjenae 2ga-gil, Seodaemun-gu, Seoul, 03641, Korea Tel: 82-2-391-0950, 396-0950 Fax: 82-2-392-2859

Name of Depository Authority: Korean Culture Center of Microorganisms (KCCM)

Accession No. : KCCM12680P

Accession date : 20200327

BUDAPEST TREATY ON THE INTERNATIONAL RECOGNITION OF THE DEPOSIT OF MICROORGANISMS FOR THE PURPOSES OF PATENT PROCEDURE

INTERNATIONAL FORM

To. COSMAX CO., LTD.
46, Jeyakgongdan 2-gil,
Hyangnam-eup. Hwaseong-si,
Gyeonggi-do 18622,
Republic of Korea

RECEIPT IN THE CASE OF AN ORIGINAL DEPOSIT issued pursuant to Rule 7.1 by the INTERNATIONAL DEPOSITARY AUTHORITY identified at the bottom of this page

| I. IDENTIFICATION OF THE MICROORGANISM | |
|---|---|
| Identification reference given by the DEPOSITOR: *Cutibacterium acnes* subsp. *acnes* YM-1 | Accession number given by the INTERNATIONAL DEPOSITARY AUTHORITY: KCCM12680P |
| II. SCIENTIFIC DESCRIPTION AND/OR PROPOSED TAXONOMIC DESIGNATION | |
| The microorganism identified under I above was accompanied by:<br>☐ a scientific description<br>☐ a proposed taxonomic designation<br>(Mark with a cross where applicable) | |
| III. RECEIPT AND ACCEPTANCE | |
| This International Depositary Authority accepts the microorganism identified under I above, which was received by it on March. 27. 2020 (date of the original deposit).[1] | |
| IV. RECEIPT OF REQUEST FOR CONVERSION | |
| The microorganism identified under I above was received by this International Depositary Authority on (date of the original deposit) and a request to convert the original deposit to a deposit under the Budapest Treaty was received by it on (date of receipt of request for conversion). | |
| V. INTERNATIONAL DEPOSITARY AUTHORITY | |
| Name : Korean Culture Center of Microorganisms<br><br>Address : Yurim B/D<br>45, Hongjenae-2ga-gil<br>Seodaemun-gu<br>SEOUL 03641<br>Republic of Korea | Signature(s) of person(s) having the power to represent the International Depositary Authority or of authorized official(s):<br><br>Date: March. 27. 2020.<br>한국미생물보존센터장의인 |

[1] Where Rule 6.4(d) applies, such date is the date on which the status of international depositary authority was acquired.

Form BP/4 (sole page)

한국미생물보존센터
03641 서울시 서대문구 홍제내2가길 45 유림빌딩 Tel 02-391-0950, 396-0950 Fax 02-392-2859
KOREAN CULTURE CENTER OF MICROORGANISMS
Yoolim Bldg., 45, Hongjenae 2ga-gil, Seodaemun-gu, Seoul, 03641, Korea Tel: 82-2-391-0950, 396-0950 Fax 82-2-392-2859

Name of Depository Authority: Korean Culture Center of Microorganisms (KCCM)

Accession No : KCCM12689P

Accession date : 20200409

BUDAPEST TREATY ON THE INTERNATIONAL RECOGNITION OF THE DEPOSIT OF MICROORGANISMS FOR THE PURPOSES OF PATENT PROCEDURE

INTERNATIONAL FORM

To. COSMAX CO., LTD.
46, Jeyakgongdan 2-gil,
Hyangnam-eup, Hwaseong-si,
Gyeonggi-do 18622,
Republic of Korea

RECEIPT IN THE CASE OF AN ORIGINAL DEPOSIT issued pursuant to Rule 7.1 by the INTERNATIONAL DEPOSITARY AUTHORITY identified at the bottom of this page

| | |
|---|---|
| I. IDENTIFICATION OF THE MICROORGANISM | |
| Identification reference given by the DEPOSITOR:<br>*Corynebacterium amycolatum* SB-1 | Accession number given by the INTERNATIONAL DEPOSITARY AUTHORITY:<br>KCCM12689P |
| II. SCIENTIFIC DESCRIPTION AND/OR PROPOSED TAXONOMIC DESIGNATION | |
| The microorganism identified under I above was accompanied by:<br>☐ a scientific description<br>☐ a proposed taxonomic designation<br>(Mark with a cross where applicable) | |
| III. RECEIPT AND ACCEPTANCE | |
| This International Depositary Authority accepts the microorganism identified under I above, which was received by it on April. 09. 2020 (date of the original deposit).[1] | |
| IV. RECEIPT OF REQUEST FOR CONVERSION | |
| The microorganism identified under I above was received by this International Depositary Authority on (date of the original deposit) and a request to convert the original deposit to a deposit under the Budapest Treaty was received by it on (date of receipt of request for conversion). | |
| V. INTERNATIONAL DEPOSITARY AUTHORITY | |
| Name : Korean Culture Center of Microorganisms<br>Address : Yurim B/D<br>45, Hongjenae-2ga-gil<br>Seodaemun-gu<br>SEOUL 03641<br>Republic of Korea | Signature(s) of person(s) having the power to represent the International Depositary Authority or of authorized official(s):<br>Date: April. 09. 2020. |

[1] Where Rule 6.4(d) applies, such date is the date on which the status of international depositary authority was acquired.

Form BP/4 (sole page)

한국미생물보존센터
03641 서울시 서대문구 홍제내2가길 45 유림빌딩 Tel: 02-391-0950, 396-0950 Fax: 02-392-2859
KOREAN CULTURE CENTER OF MICROORGANISMS
Yoolim Bldg., 45, Hongjenae 2ga-gil, Seodaemun-gu, Seoul, 03641, Korea Tel: 82-2-391-0950, 396-0950 Fax: 82-2-392-2859

Name of Depository Authority: Korean Culture Center of Microorganisms (KCCM)

Accession No. : KCCM12685P

Accession date : 20200327

BUDAPEST TREATY ON THE INTERNATIONAL RECOGNITION OF THE DEPOSIT OF MICROORGANISMS FOR THE PURPOSES OF PATENT PROCEDURE

INTERNATIONAL FORM

To. COSMAX CO., LTD.
46, Jeyakgongdan 2-gil,
Hyangnam-eup, Hwaseong-si,
Gyeonggi-do 18622,
Republic of Korea

RECEIPT IN THE CASE OF AN ORIGINAL DEPOSIT issued pursuant to Rule 7.1 by the INTERNATIONAL DEPOSITARY AUTHORITY identified at the bottom of this page

| I. IDENTIFICATION OF THE MICROORGANISM | |
|---|---|
| Identification reference given by the DEPOSITOR: *Rothia kristinae* YM-6 | Accession number given by the INTERNATIONAL DEPOSITARY AUTHORITY: KCCM12685P |
| II. SCIENTIFIC DESCRIPTION AND/OR PROPOSED TAXONOMIC DESIGNATION | |
| The microorganism identified under I above was accompanied by:<br>☐ a scientific description<br>☐ a proposed taxonomic designation<br>(Mark with a cross where applicable) | |
| III. RECEIPT AND ACCEPTANCE | |
| This International Depositary Authority accepts the microorganism identified under I above, which was received by it on March. 27. 2020 (date of the original deposit).[1] | |
| IV. RECEIPT OF REQUEST FOR CONVERSION | |
| The microorganism identified under I above was received by this International Depositary Authority on (date of the original deposit) and a request to convert the original deposit to a deposit under the Budapest Treaty was received by it on (date of receipt of request for conversion). | |
| V. INTERNATIONAL DEPOSITARY AUTHORITY | |
| Name : Korean Culture Center of Microorganisms<br>Address : Yurim B/D<br>45, Hongjenae-2ga-gil<br>Seodaemun-gu<br>SEOUL 03641<br>Republic of Korea | Signature(s) of person(s) having the power to represent the International Depositary Authority or of authorized official(s):<br>Date: March. 27. 2020 |

[1] Where Rule 6.4(d) applies, such date is the date on which the status of international depositary authority was acquired.

Form BP/4 (sole page)

한국미생물보존센터
03641 서울시 서대문구 홍제내2가길 45 유림빌딩 Tel. 02-391-0950, 396-0950 Fax. 02-392-2859
KOREAN CULTURE CENTER OF MICROORGANISMS
Yoolim Bldg., 45, Hongjenae 2ga-gil, Seodaemun-gu, Seoul, 03641, Korea Tel: 82-2-391-0950, 396-0950 Fax: 82-2-392-2859

## Claims

1. A medium composition for promoting growth of microorganisms, the medium composition comprising: glucose, fructose and rhamnose as sugars; threonine, tyrosine, arginine, alanine, proline, histidine, iso-leucine, leucine, phenyl-alanine, tryptophan, valine, glutamic acid, aspartic acid, serine and glycine as amino acids.

2. The medium composition of claim 1, wherein the sugars, the amino acids, and the microorganisms are derived from human skin.

3. The medium composition of claim 2, wherein the microorganisms are at least one selected from the group consisting of *Staphylococcus epidermidis* KCCM12551P, *Streptococcus thermophilus* KCCM12004P, *Cutibacterium acnes* KCCM12680P, *Corynebacterium amycolatum* KCCM12689P, and *Rothia kristinae* KCCM12685P.

4. The medium composition of claim 1, further comprising glycerol, magnesium sulfate ($MgSO_4$), potassium phosphate ($K_2HPO_4$), sodium chloride (NaCl), and a yeast extract.

5. A method of preparing a cosmetic composition for skin improvement, the method comprising: inoculating and culturing each of microorganisms of the genus *Staphylococcus,* the genus *Streptococcus,* the genus *Cutibacterium,* the genus *Corynebacterium,* or the genus *Rothia,* in the medium composition of claim 1, to prepare a culture solution;

centrifuging and filtering each culture solution to collect a supernatant of each culture solution; and
mixing each supernatant.

6. The method of claim 5, wherein the skin improvement comprises skin moisturization, skin barrier strengthening, skin whitening, wrinkle reduction, skin texture improvement, pore reduction, skin tone improvement, skin elasticity improvement, or skin density improvement.

7. The method of claim 5, wherein the microorganism of the genus *Staphylococcus* is *Staphylococcus epidermidis* KCCM12551P, the microorganism of the genus *Streptococcus* is *Streptococcus thermophilus* KCCM12004P, the microorganism of the genus *Cutibacterium* is *Cutibacterium acnes* KCCM12680P, the microorganism of the genus *Corynebacterium* is *Corynebacterium amycolatum* KCCM12689P, and the microorganism of the genus *Rothia* is *Rothia kristinae* KCCM12685P.

8. A cosmetic composition for skin improvement, prepared by the method of any one of claims 5 to 7.

9. The cosmetic composition of claim 8, wherein the cosmetic composition comprises microorganisms of the genus *Staphylococcus,* the genus *Streptococcus,* the genus *Cutibacterium,* the genus *Corynebacterium* or the genus *Rothia,* a culture solution thereof, or a supernatant of the culture solution.

10. The cosmetic composition of claim 9, wherein the microorganism of the genus *Staphylococcus* is *Staphylococcus epidermidis* KCCM12551P, the microorganism of the genus *Streptococcus* is *Streptococcus thermophilus* KCCM12004P, the microorganism of the genus *Cutibacterium* is *Cutibacterium acnes* KCCM12680P, the microorganism of the genus *Corynebacterium* is *Corynebacterium amycolatum* KCCM12689P, and the microorganism of the genus *Rothia* is *Rothia kristinae* KCCM12685P.

11. The cosmetic composition of claim 8, wherein the skin improvement comprises skin moisturization, skin barrier strengthening, skin whitening, wrinkle reduction, skin texture improvement, pore reduction, skin tone improvement, skin elasticity improvement, or skin density improvement.

12. The cosmetic composition of claim 9, wherein the culture solution is cultured in the medium composition of any one of claims 1 to 4.

# FIG. 1

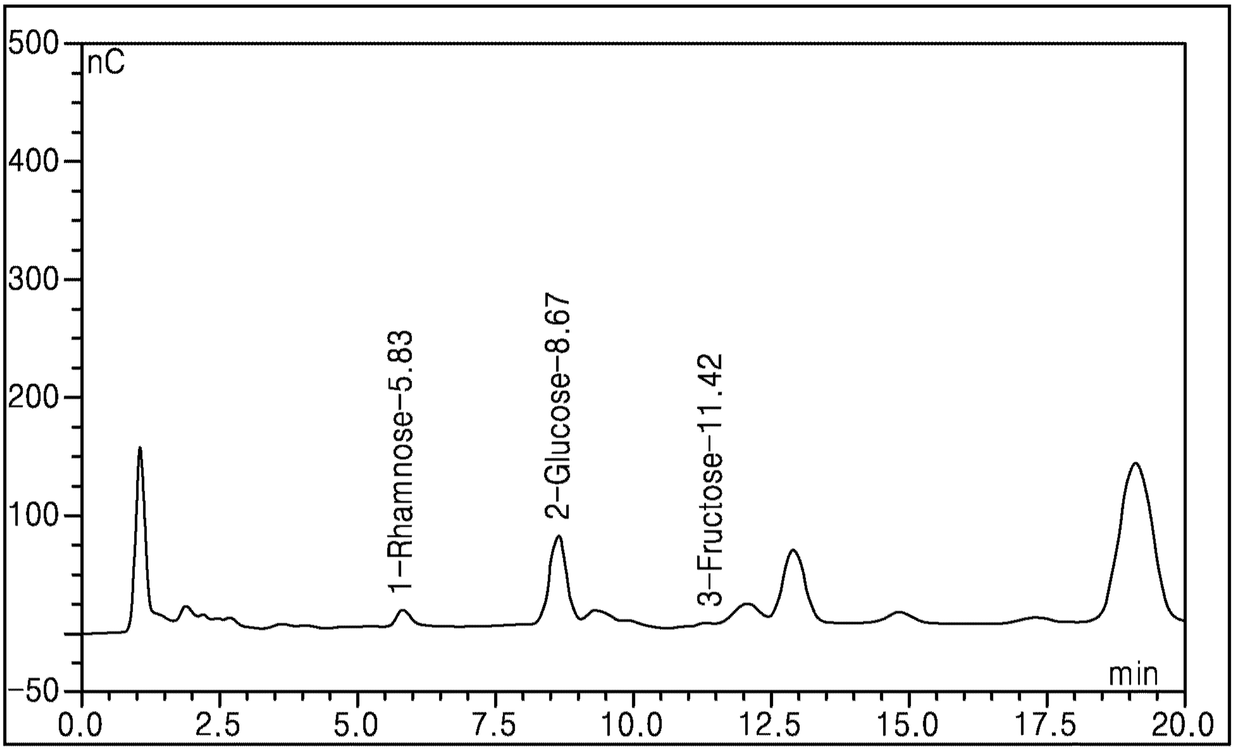


| No. | Ret.Time min | Peak Name | Height nC | Area nC*min | Rel.Area % | Amount (mg/mL) |
|---|---|---|---|---|---|---|
| 1 | 5.83 | Rhamnose | 14.596 | 4.249 | 13.24 | 0.003 |
| 2 | 8.67 | Glucose | 75.012 | 26.367 | 82.18 | 0.016 |
| 3 | 11.42 | Fluctose | 3.496 | 1.469 | 4.58 | 0.002 |
| Total : | | | 93.104 | 32.086 | 100.0 | 0.022 |

FIG. 2

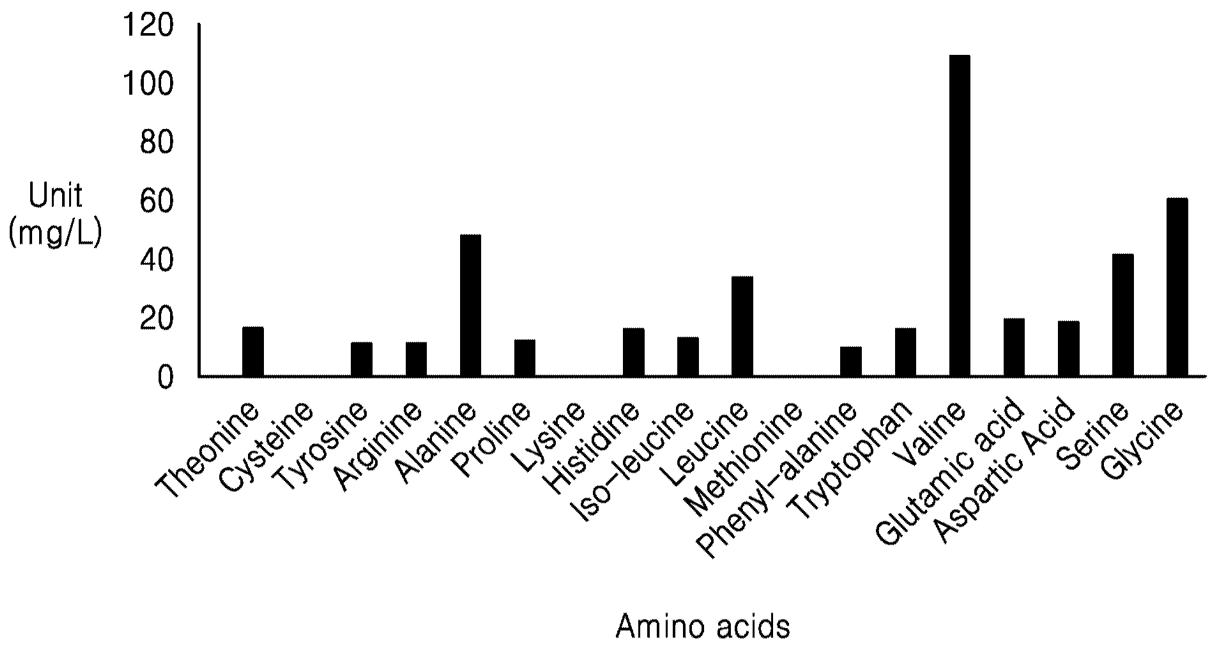
120
100
80
60
40
20
0
Unit
(mg/L)
Theonine
Cysteine
Tyrosine
Arginine
Alanine
Proline
Lysine
Histidine
Iso-leucine
Leucine
Methionine
Phenyl-alanine
Tryptophan
Valine
Glutamic acid
Aspartic Acid
Serine
Glycine
Amino acids

FIG. 3

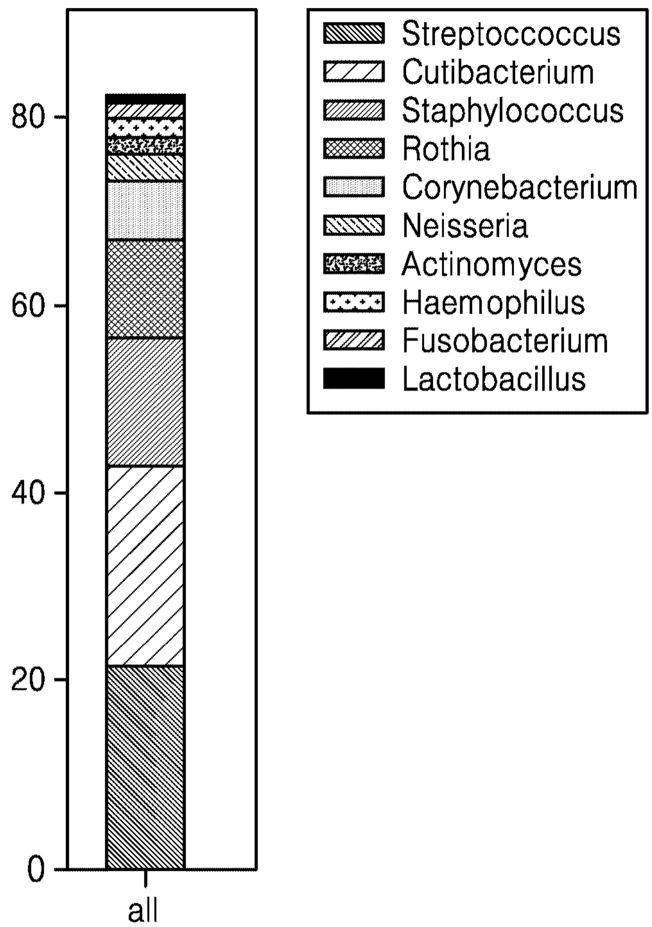
80
60
40
20
0
all
Streptoccoccus
Cutibacterium
Staphylococcus
Rothia
Corynebacterium
Neisseria
Actinomyces
Haemophilus
Fusobacterium
Lactobacillus

FIG. 4

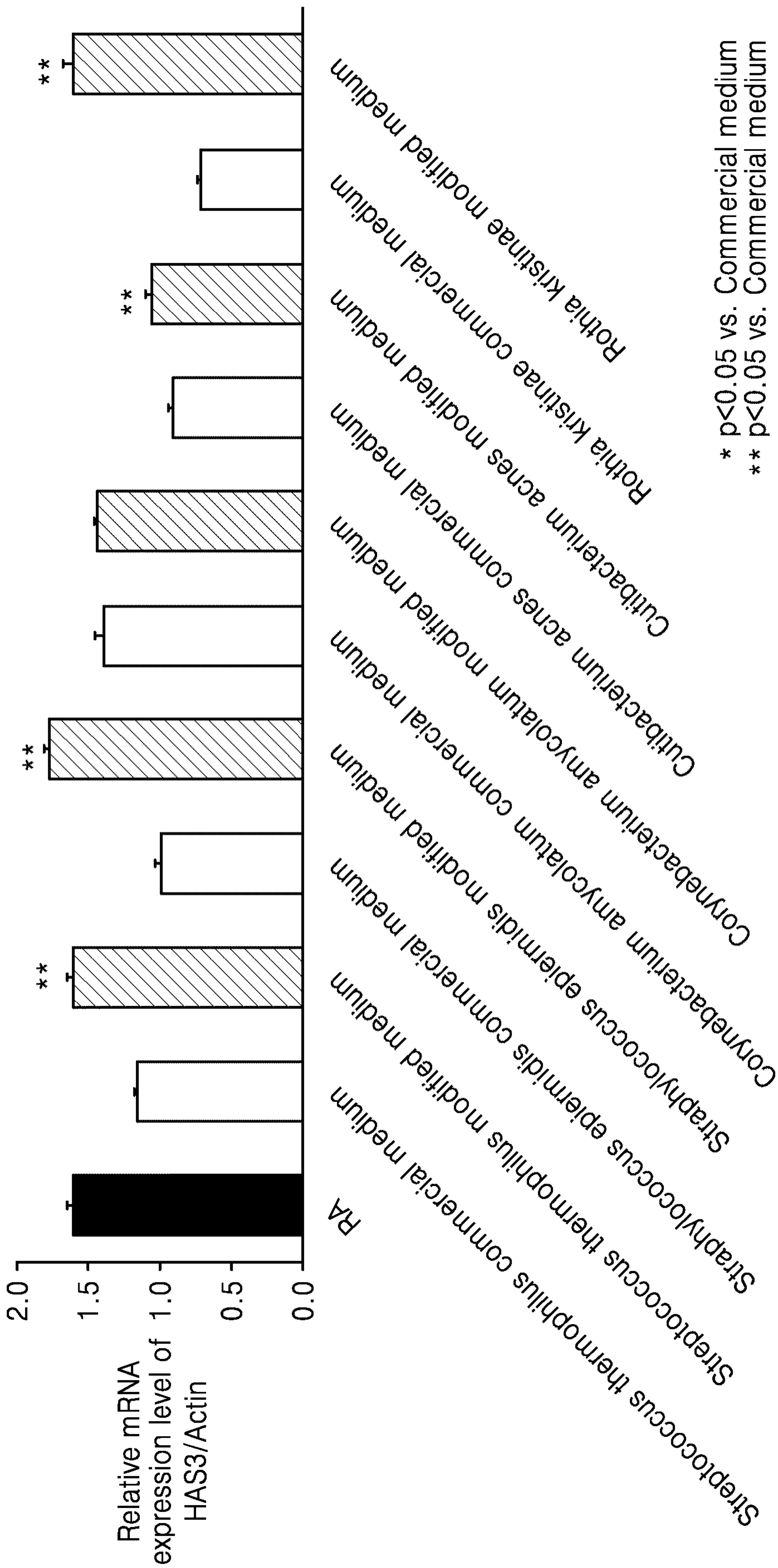
Relative mRNA expression level of HAS3/Actin
2.0
1.5
1.0
0.5
0.0
**
**
**
**
RA
Streptococcus thermophilus commercial medium
Streptococcus thermophilus modified medium
Staphylococcus epiermidis commercial medium
Staphylococcus epiermidis modified medium
Corynebacterium amycolatum commercial medium
Corynebacterium amycolatum modified medium
Cutibacterium acnes commercial medium
Cutibacterium acnes modified medium
Rothia kristinae commercial medium
Rothia kristinae modified medium
* p<0.05 vs. Commercial medium
** p<0.05 vs. Commercial medium

FIG. 5

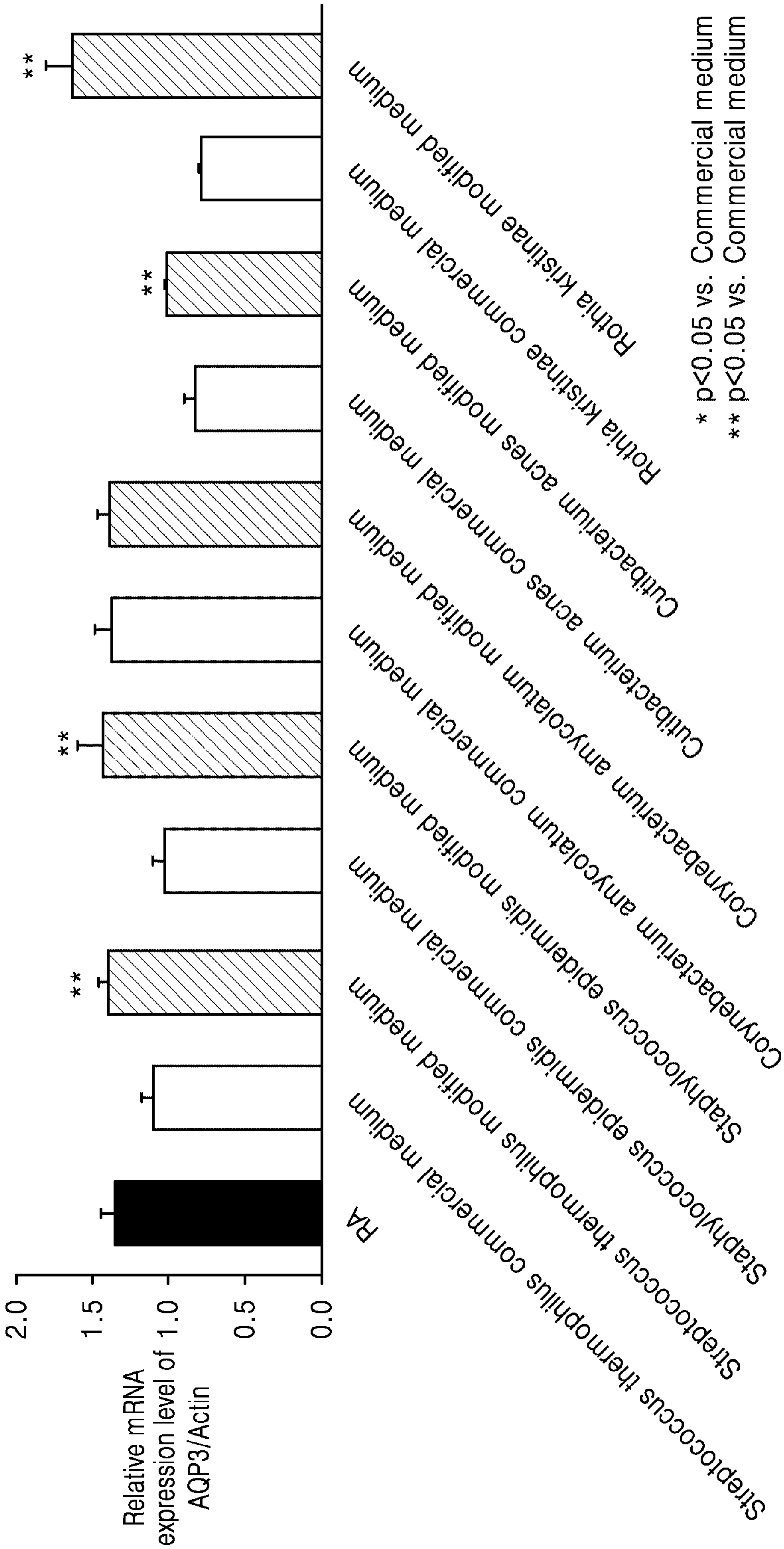
Relative mRNA expression level of AQP3/Actin
2.0
1.5
1.0
0.5
0.0
RA
Streptococcus thermophilus commercial medium
Streptococcus thermophilus modified medium
Staphylococcus epidermidis commercial medium
Staphylococcus epidermidis modified medium
Corynebacterium amycolatum commercial medium
Corynebacterium amycolatum modified medium
Cutibacterium acnes commercial medium
Cutibacterium acnes modified medium
Rothia kristinae commercial medium
Rothia kristinae modified medium
**
* p<0.05 vs. Commercial medium
** p<0.05 vs. Commercial medium

FIG. 6

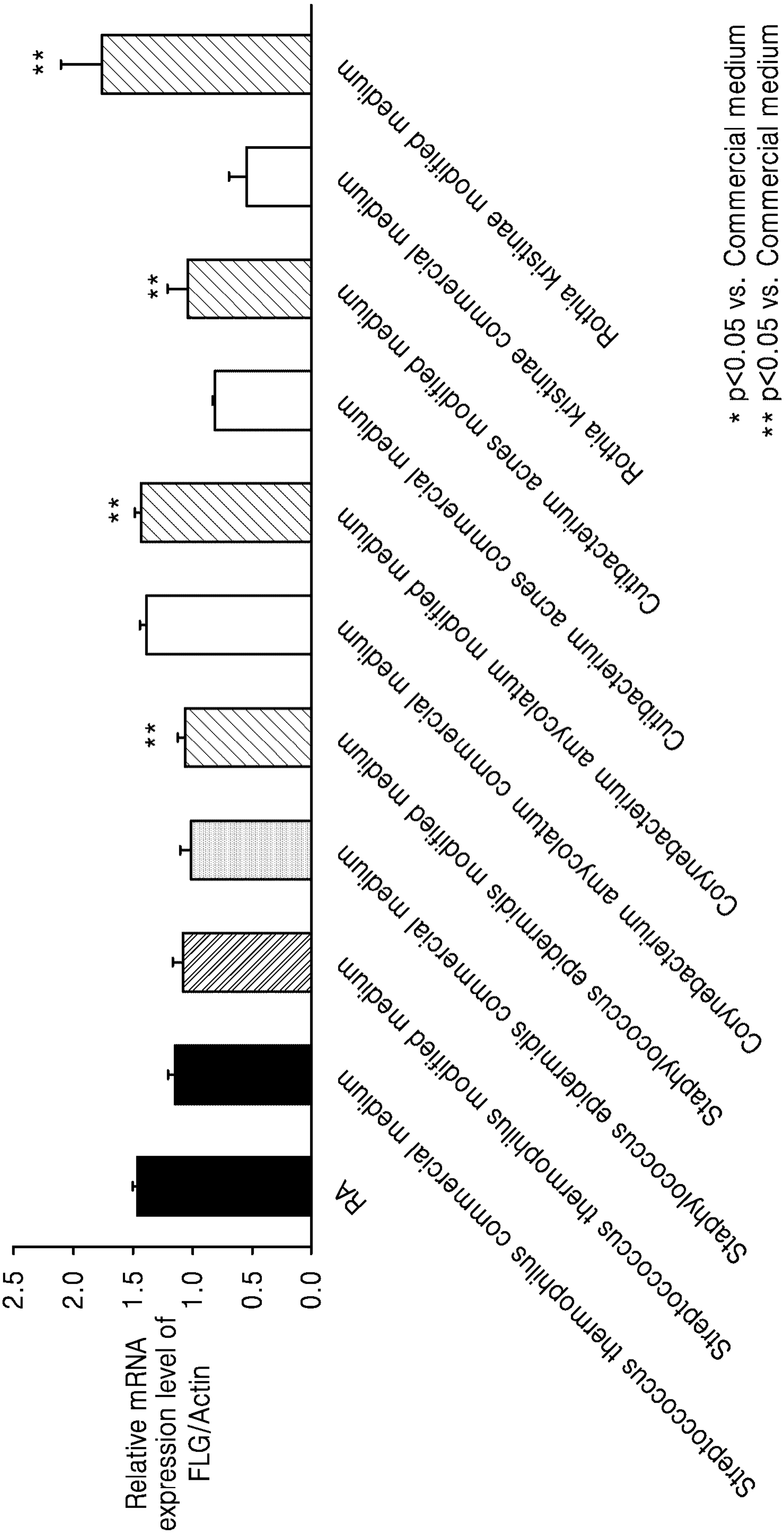
Relative mRNA expression level of FLG/Actin
0.0
0.5
1.0
1.5
2.0
2.5
RA
Streptococcus thermophilus commercial medium
Streptococcus thermophilus modified medium
Staphylococcus epidermidis commercial medium
Staphylococcus epidermidis modified medium
Corynebacterium amycolatum commercial medium
Corynebacterium amycolatum modified medium
Cutibacterium acnes commercial medium
Cutibacterium acnes modified medium
Rothia kristinae commercial medium
Rothia kristinae modified medium
**
* p<0.05 vs. Commercial medium
** p<0.05 vs. Commercial medium

FIG. 7

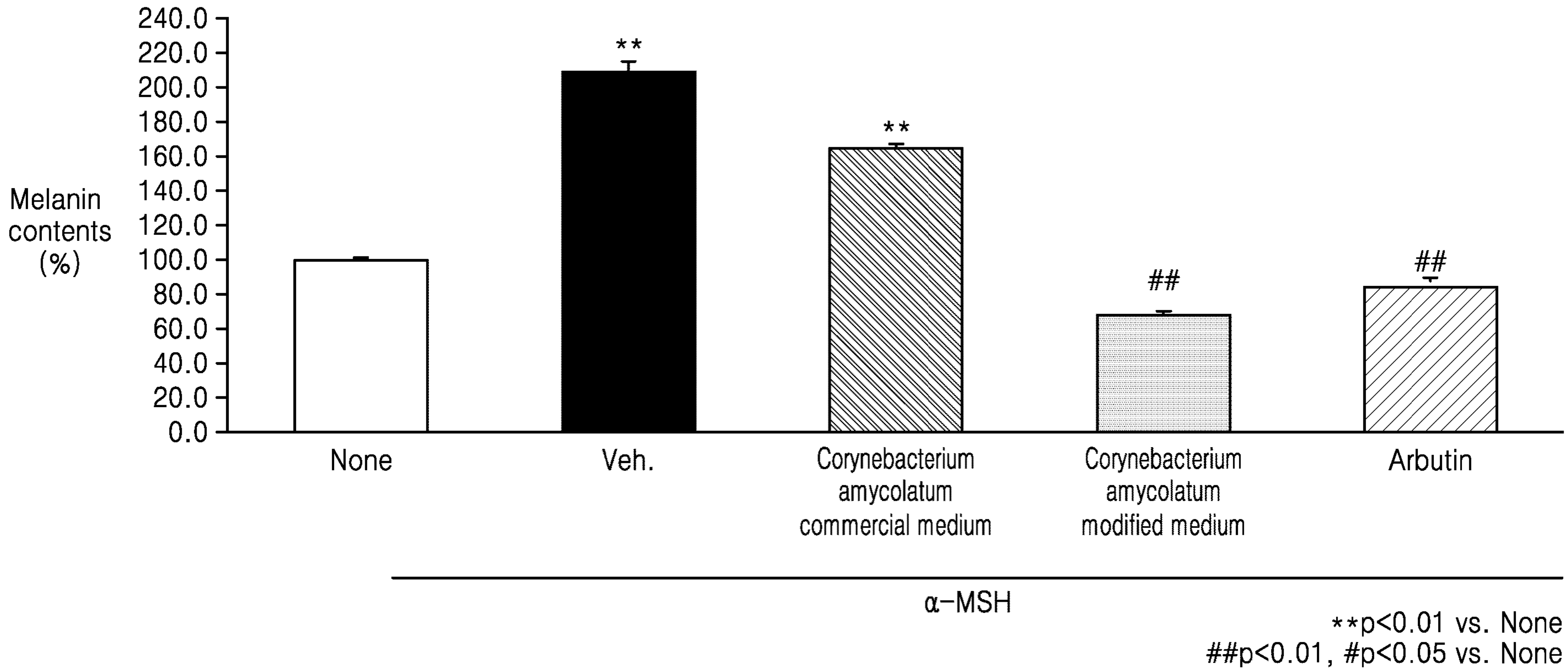
Melanin contents (%)
240.0
220.0
200.0
180.0
160.0
140.0
120.0
100.0
80.0
60.0
40.0
20.0
0.0
**
**
##
##
None
Veh.
Corynebacterium amycolatum commercial medium
Corynebacterium amycolatum modified medium
Arbutin
α−MSH
**p<0.01 vs. None
##p<0.01, #p<0.05 vs. None

FIG. 8

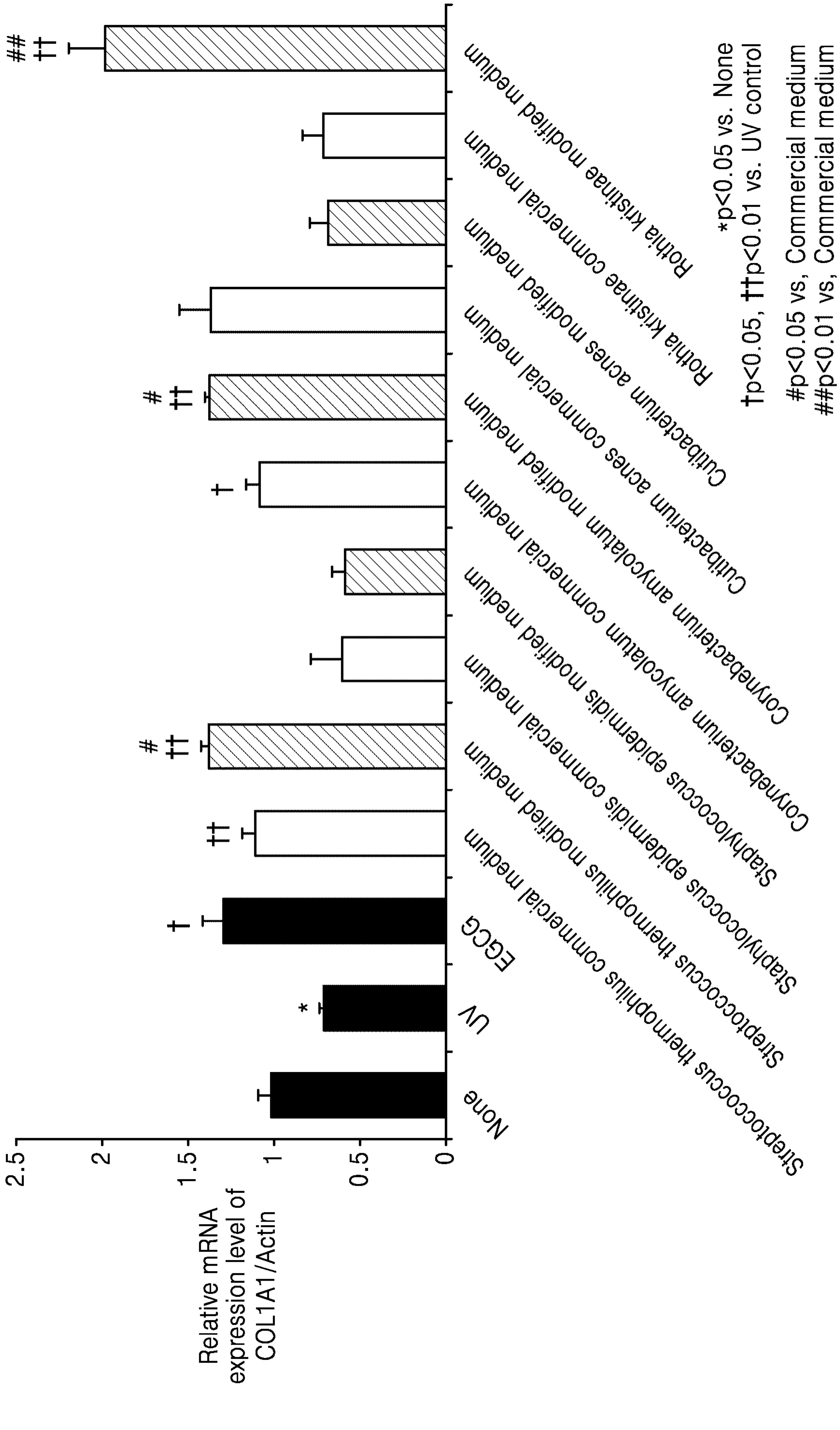
Relative mRNA expression level of COL1A1/Actin
0
0.5
1
1.5
2
2.5
None
UV
EGCG
Streptococcus thermophilus commercial medium
Streptococcus thermophilus modified medium
Staphylococcus epidermidis commercial medium
Staphylococcus epidermidis modified medium
Corynebacterium amycolatum commercial medium
Corynebacterium amycolatum modified medium
Cutibacterium acnes commercial medium
Cutibacterium acnes modified medium
Rothia kristinae commercial medium
Rothia kristinae modified medium
*
†
††
††
#
†
††
#
††
##
*p<0.05 vs. None
†p<0.05, ††p<0.01 vs. UV control
#p<0.05 vs, Commercial medium
##p<0.01 vs, Commercial medium

FIG. 9

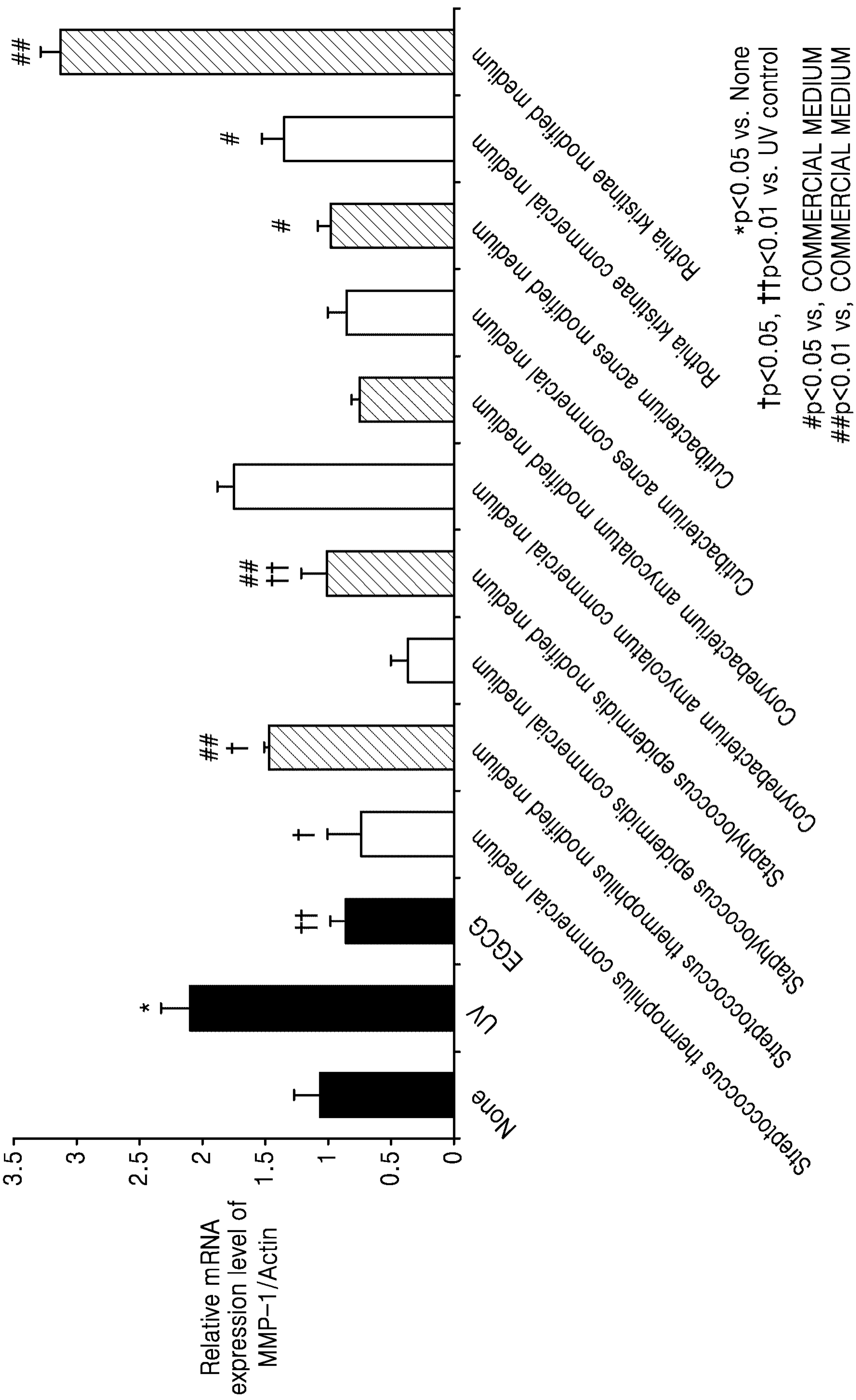
Relative mRNA expression level of MMP-1/Actin
0
0.5
1
1.5
2
2.5
3
3.5
None
UV
EGCG
Streptococcus thermophilus commercial medium
Streptococcus thermophilus modified medium
Staphylococcus epidermidis commercial medium
Staphylococcus epidermidis modified medium
Corynebacterium amycolatum commercial medium
Corynebacterium amycolatum modified medium
Cutibacterium acnes commercial medium
Cutibacterium acnes modified medium
Rothia kristinae commercial medium
Rothia kristinae modified medium
*
††
†
##
†
##
††
#
#
##
*p<0.05 vs. None
†p<0.05, ††p<0.01 vs. UV control
#p<0.05 vs, COMMERCIAL MEDIUM
##p<0.01 vs, COMMERCIAL MEDIUM

# FIG. 10

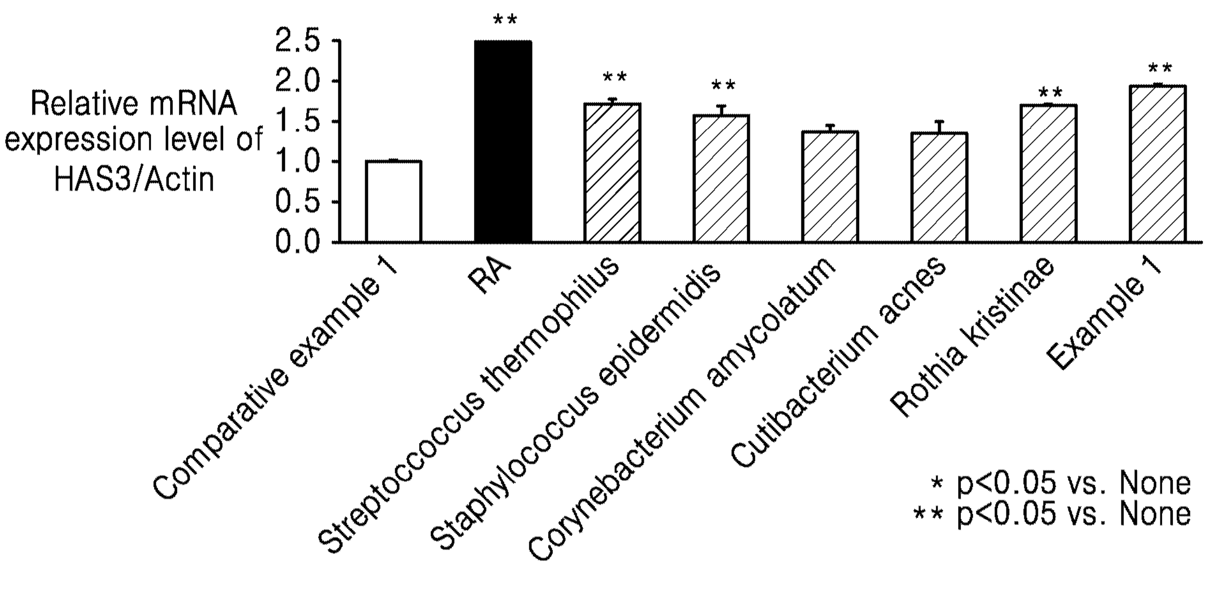
Relative mRNA expression level of HAS3/Actin
2.5
2.0
1.5
1.0
0.5
0.0
**
**
**
**
**
Comparative example 1
RA
Streptoccoccus thermophilus
Staphylococcus epidermidis
Corynebacterium amycolatum
Cutibacterium acnes
Rothia kristinae
Example 1
* p<0.05 vs. None
** p<0.05 vs. None

# FIG. 11

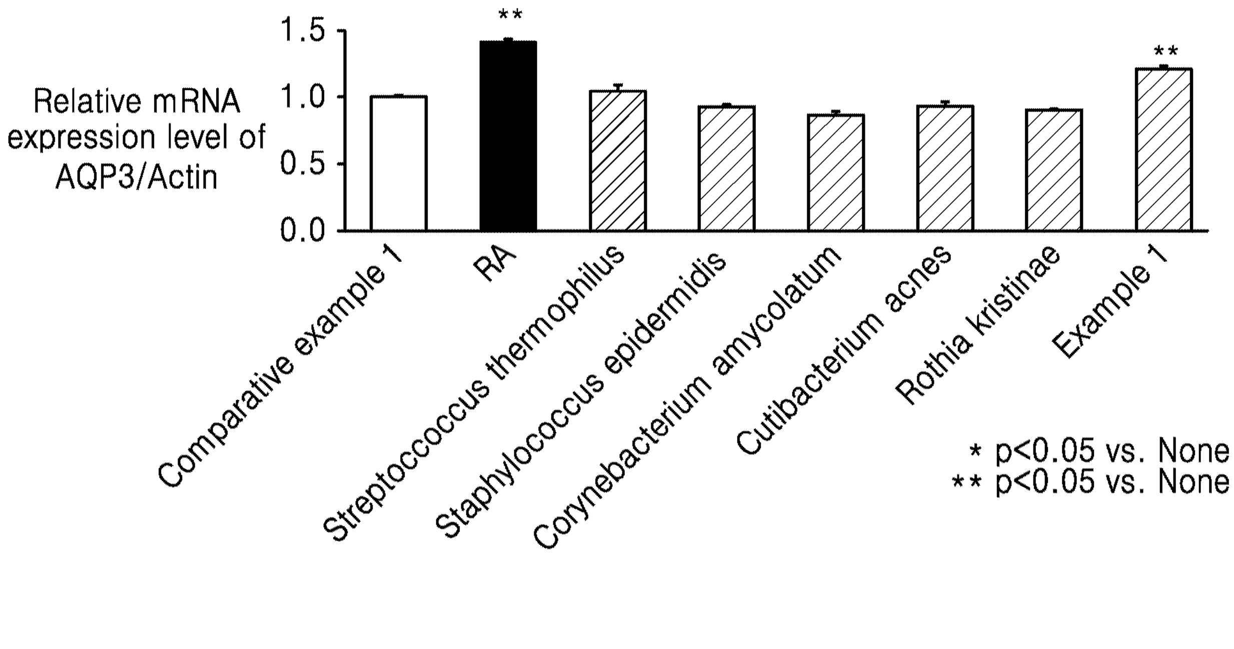

Relative mRNA expression level of AQP3/Actin
1.5
1.0
0.5
0.0
**
**
Comparative example 1
RA
Streptococcus thermophilus
Staphylococcus epidermidis
Corynebacterium amycolatum
Cutibacterium acnes
Rothia kristinae
Example 1
* p<0.05 vs. None
** p<0.05 vs. None

FIG. 12

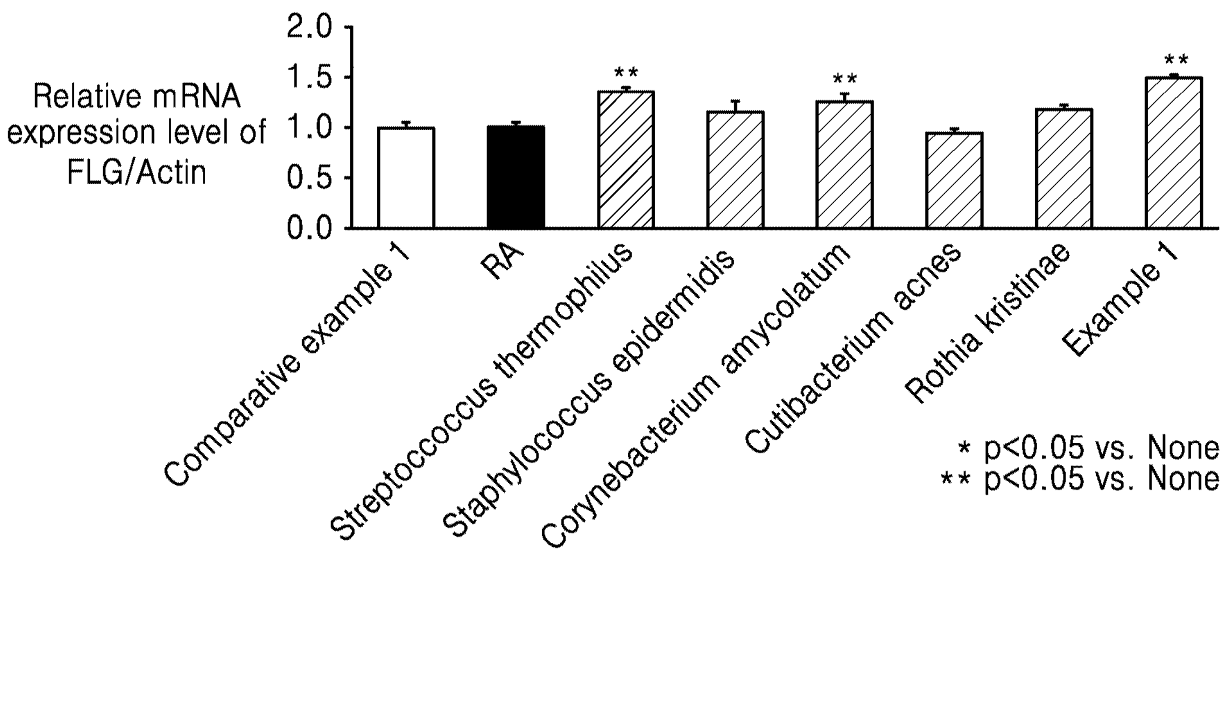
Relative mRNA expression level of FLG/Actin
2.0
1.5
1.0
0.5
0.0
**
**
**
Comparative example 1
RA
Streptococcus thermophilus
Staphylococcus epidermidis
Corynebacterium amycolatum
Cutibacterium acnes
Rothia kristinae
Example 1
* p<0.05 vs. None
** p<0.05 vs. None

FIG. 13

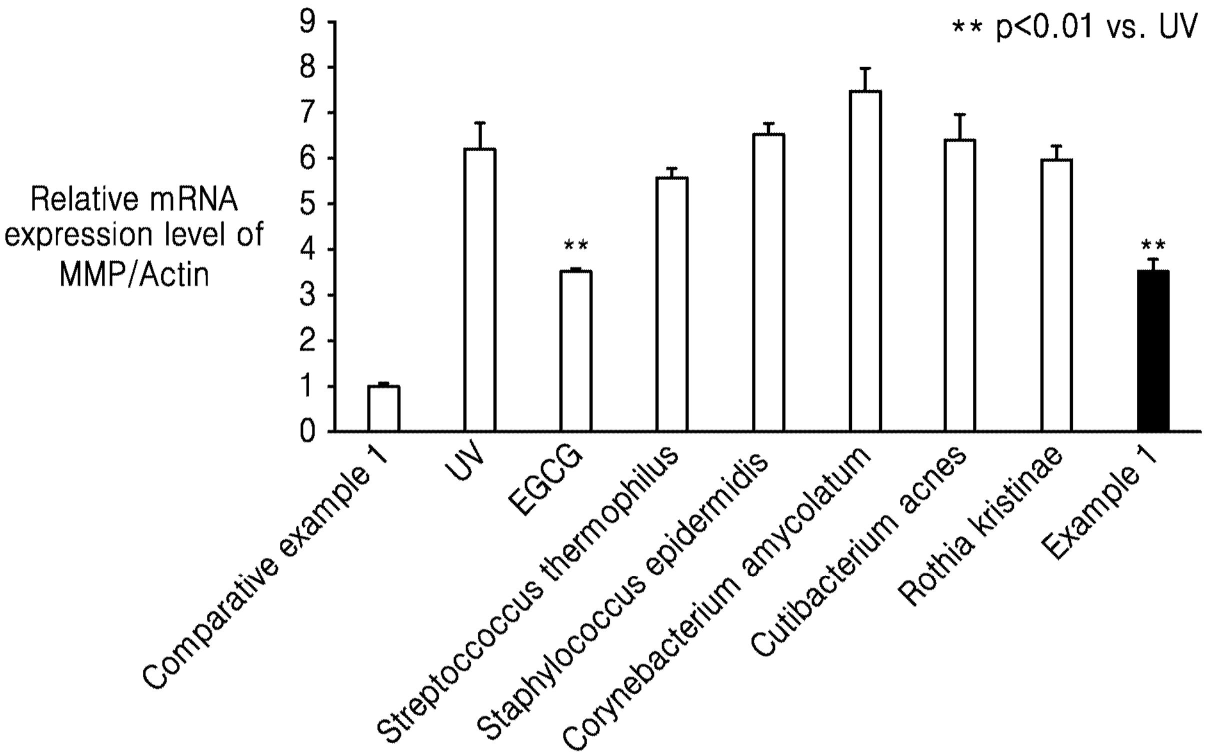
** p<0.01 vs. UV
Relative mRNA expression level of MMP/Actin
9
8
7
6
5
4
3
2
1
0
**
**
Comparative example 1
UV
EGCG
Streptococcus thermophilus
Staphylococcus epidermidis
Corynebacterium amycolatum
Cutibacterium acnes
Rothia kristinae
Example 1

FIG. 14

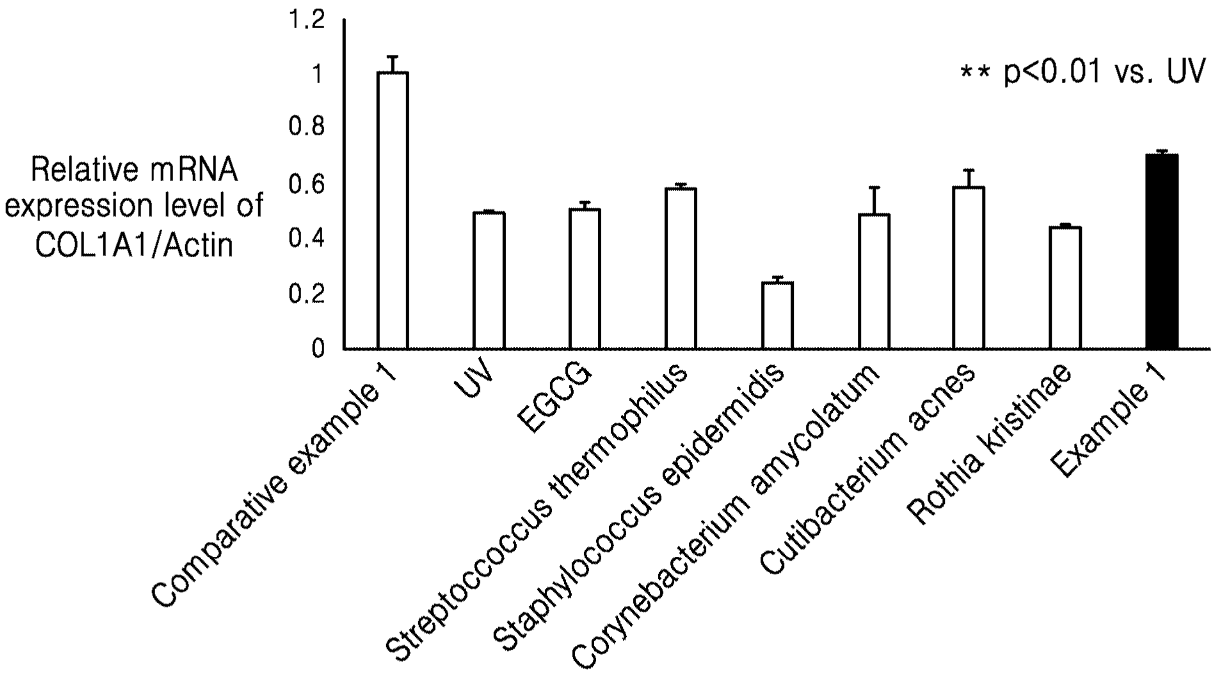
1.2
1
0.8
0.6
0.4
0.2
0
Relative mRNA expression level of COL1A1/Actin
** p<0.01 vs. UV
Comparative example 1
UV
EGCG
Streptococcus thermophilus
Staphylococcus epidermidis
Corynebacterium amycolatum
Cutibacterium acnes
Rothia kristinae
Example 1

FIG. 15

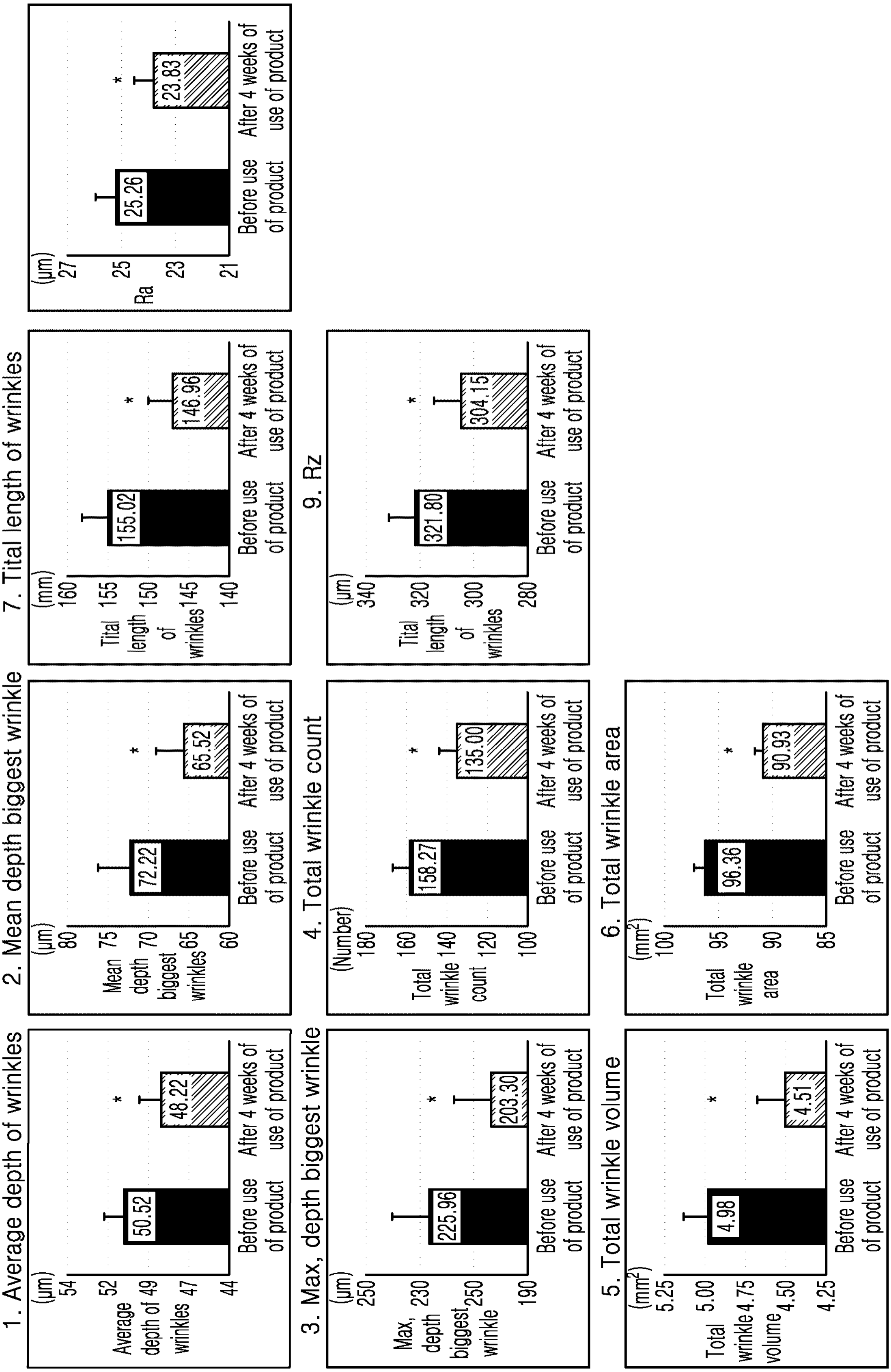
1. Average depth of wrinkles
(µm)
54
52
Average depth of 49 wrinkles
47
44
50.52
48.22
*
Before use of product
After 4 weeks of use of product
2. Mean depth biggest wrinkle
(µm)
80
75
70
65
60
Mean depth biggest wrinkles
72.22
65.52
7. Tital length of wrinkles
(mm)
160
155
150
145
140
Tital length of wrinkles
155.02
146.96
(µm)
27
25
23
21
Ra
25.26
23.83
3. Max, depth biggest wrinkle
(µm)
250
230
250
190
Max, depth biggest wrinkle
225.96
203.30
4. Total wrinkle count
(Number)
180
160
140
120
100
Total wrinkle count
158.27
135.00
9. Rz
(µm)
340
320
300
280
Tital length of wrinkles
321.80
304.15
5. Total wrinkle volume
(mm³)
5.25
5.00
4.75
4.50
4.25
Total wrinkle volume
4.98
4.51
6. Total wrinkle area
(mm²)
100
95
90
85
Total wrinkle area
96.36
90.93

FIG. 16

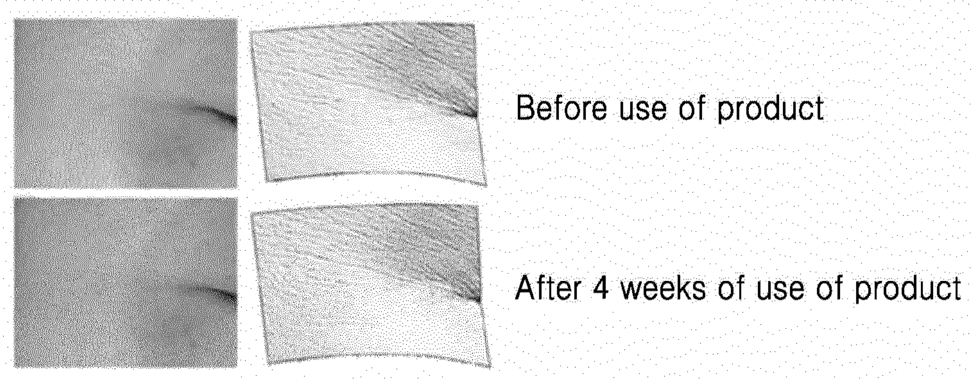

FIG. 17

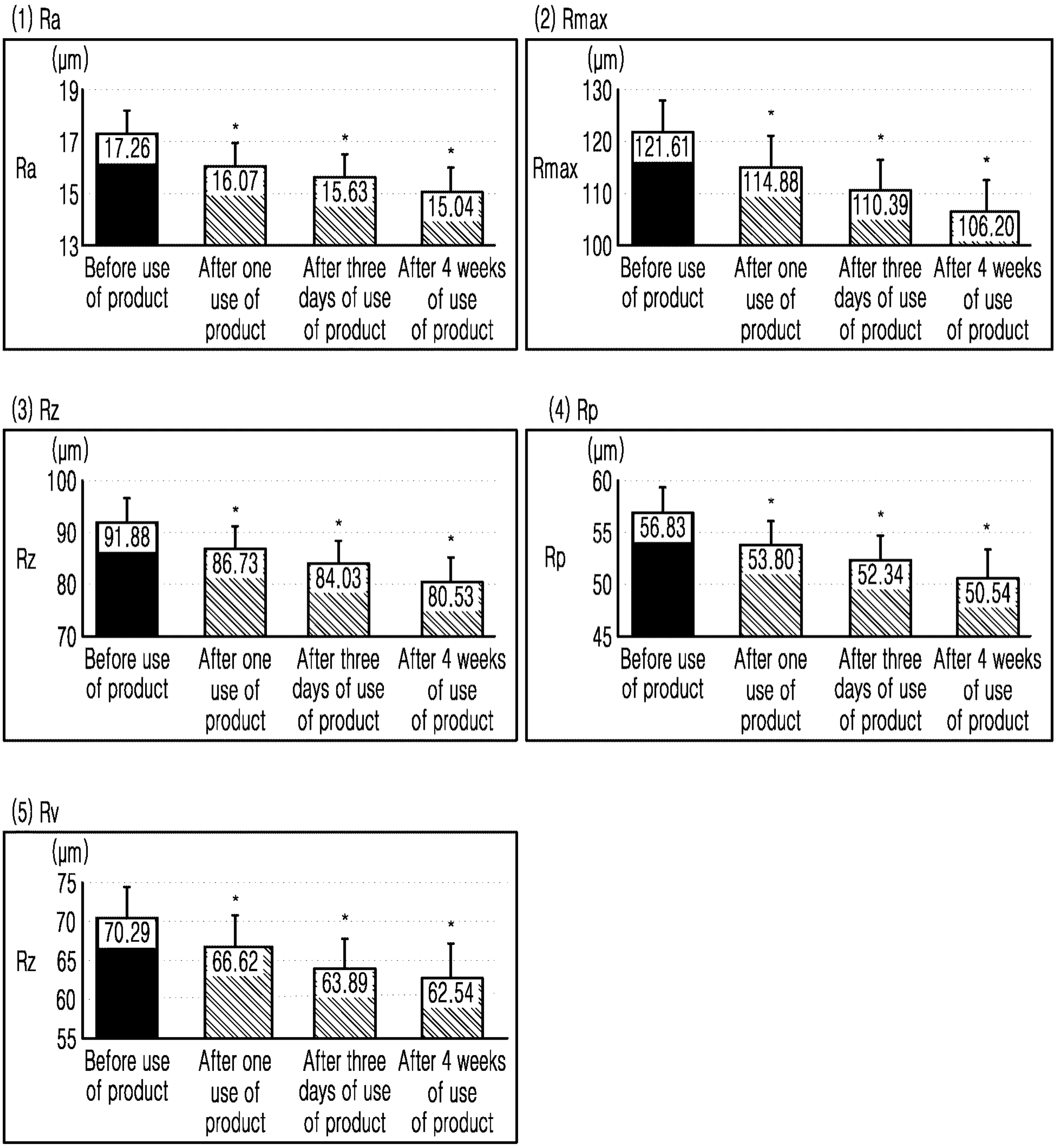
(1) Ra
(μm)
19
17
15
13
Ra
17.26
16.07
15.63
15.04
*
Before use of product
After one use of product
After three days of use of product
After 4 weeks of use of product
(2) Rmax
(μm)
130
120
110
100
Rmax
121.61
114.88
110.39
106.20
*
Before use of product
After one use of product
After three days of use of product
After 4 weeks of use of product
(3) Rz
(μm)
100
90
80
70
Rz
91.88
86.73
84.03
80.53
*
Before use of product
After one use of product
After three days of use of product
After 4 weeks of use of product
(4) Rp
(μm)
60
55
50
45
Rp
56.83
53.80
52.34
50.54
*
Before use of product
After one use of product
After three days of use of product
After 4 weeks of use of product
(5) Rv
(μm)
75
70
65
60
55
Rz
70.29
66.62
63.89
62.54
*
Before use of product
After one use of product
After three days of use of product
After 4 weeks of use of product

FIG. 18

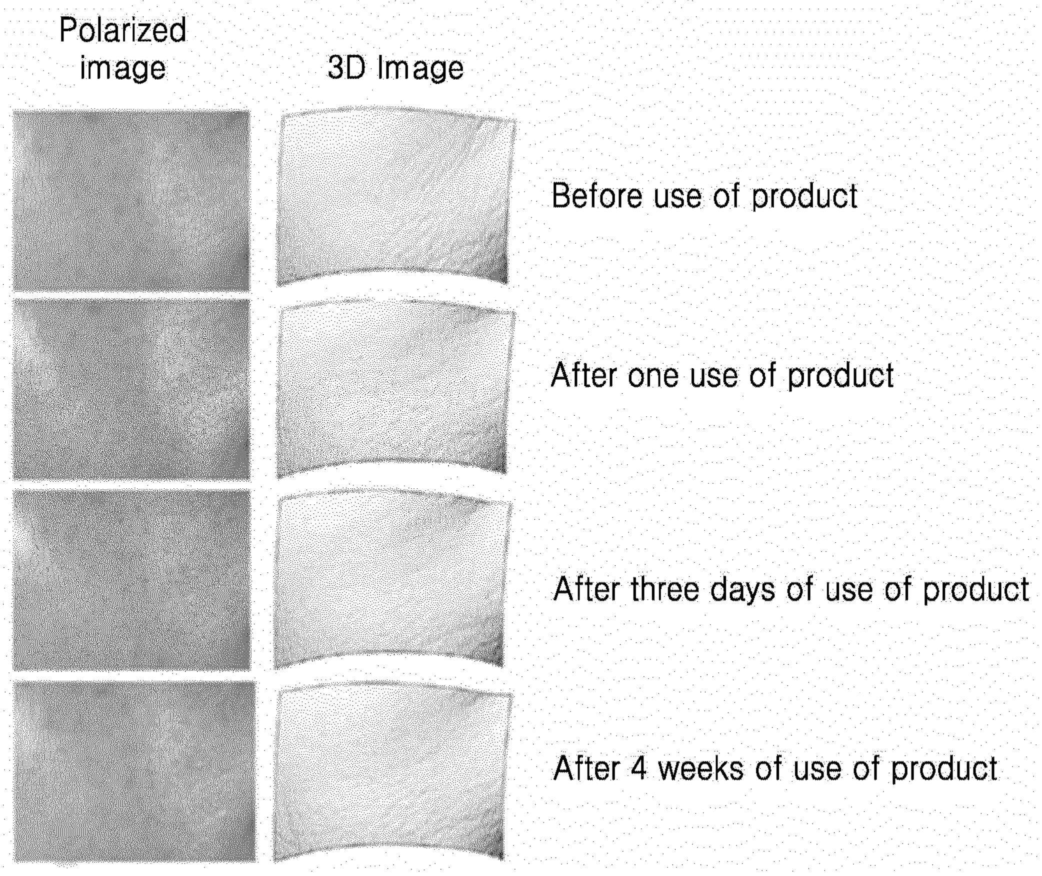
Polarized image
3D Image
Before use of product
After one use of product
After three days of use of product
After 4 weeks of use of product

# FIG. 19

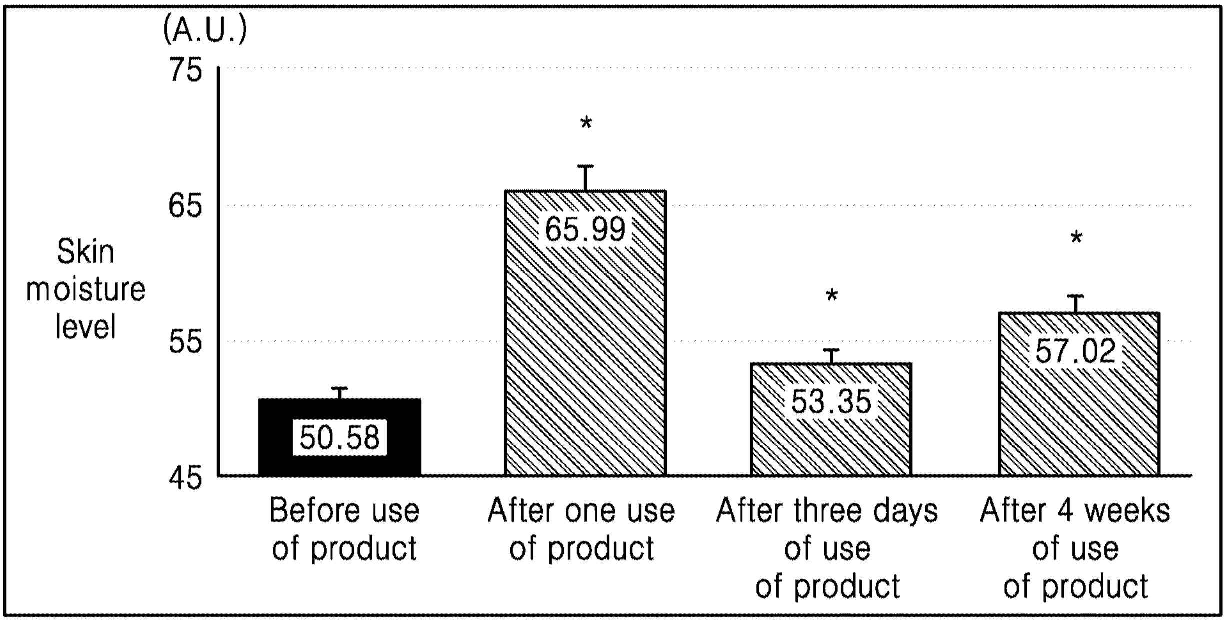
(A.U.)
75
65
55
45
Skin moisture level
*
65.99
*
53.35
*
57.02
50.58
Before use of product
After one use of product
After three days of use of product
After 4 weeks of use of product

FIG. 20

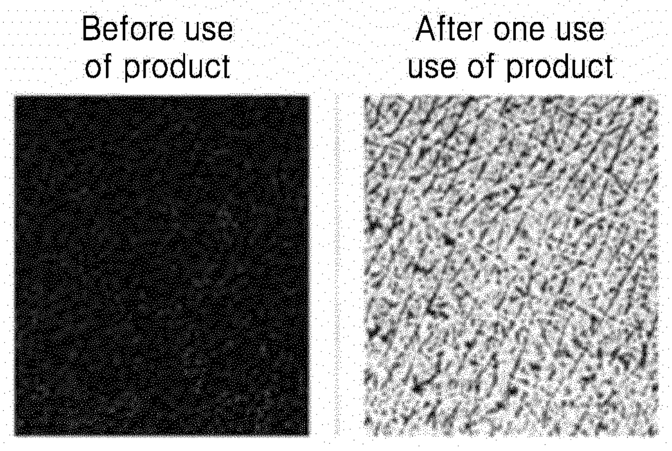

FIG. 21

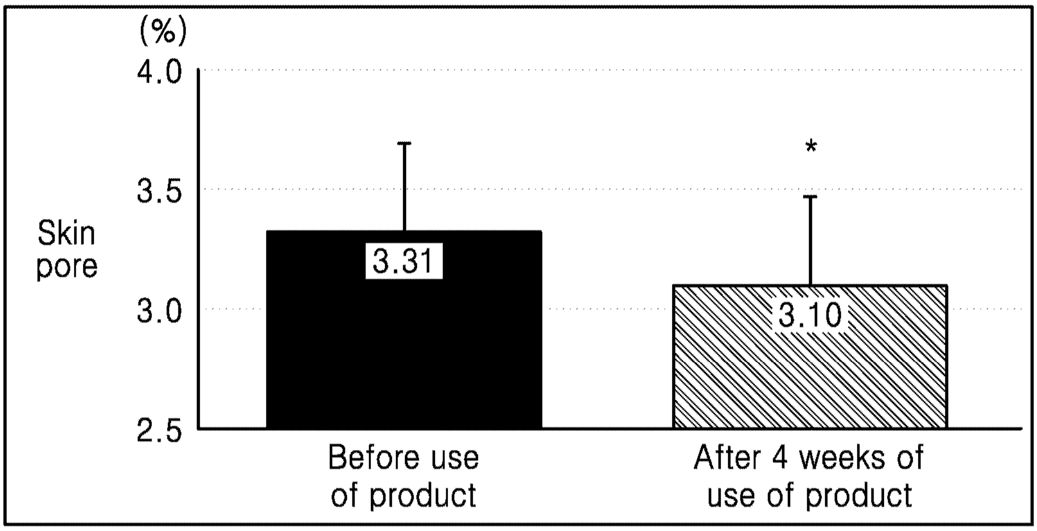
(%)
4.0
3.5
3.0
2.5
Skin pore
3.31
3.10
*
Before use of product
After 4 weeks of use of product

FIG. 22

Before use of product

After 4 weeks of use of product

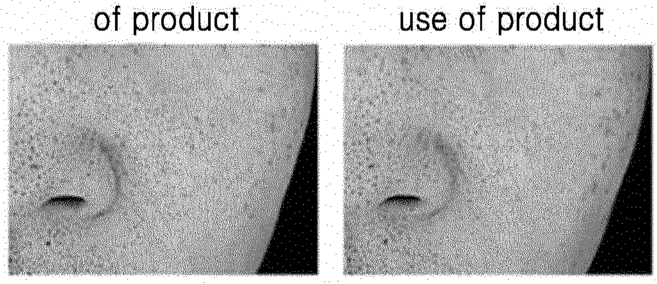

FIG. 23

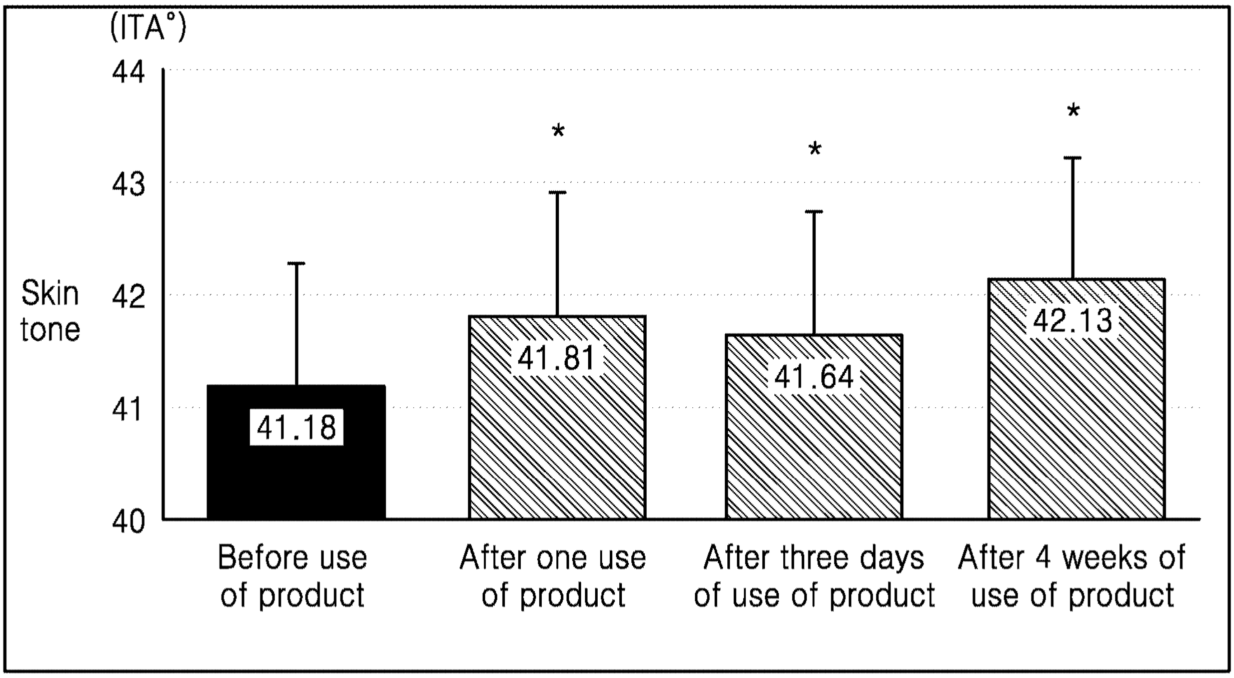
(ITA°)
44
43
42
41
40
Skin tone
*
*
*
41.18
41.81
41.64
42.13
Before use of product
After one use of product
After three days of use of product
After 4 weeks of use of product

FIG. 24

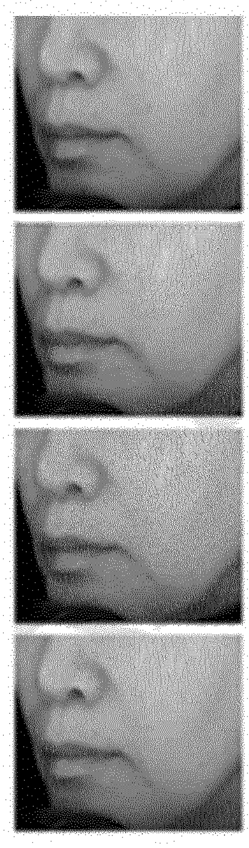

Before use of product

After one use of product

After three days of use of product

After 4 weeks of use of product

FIG. 25

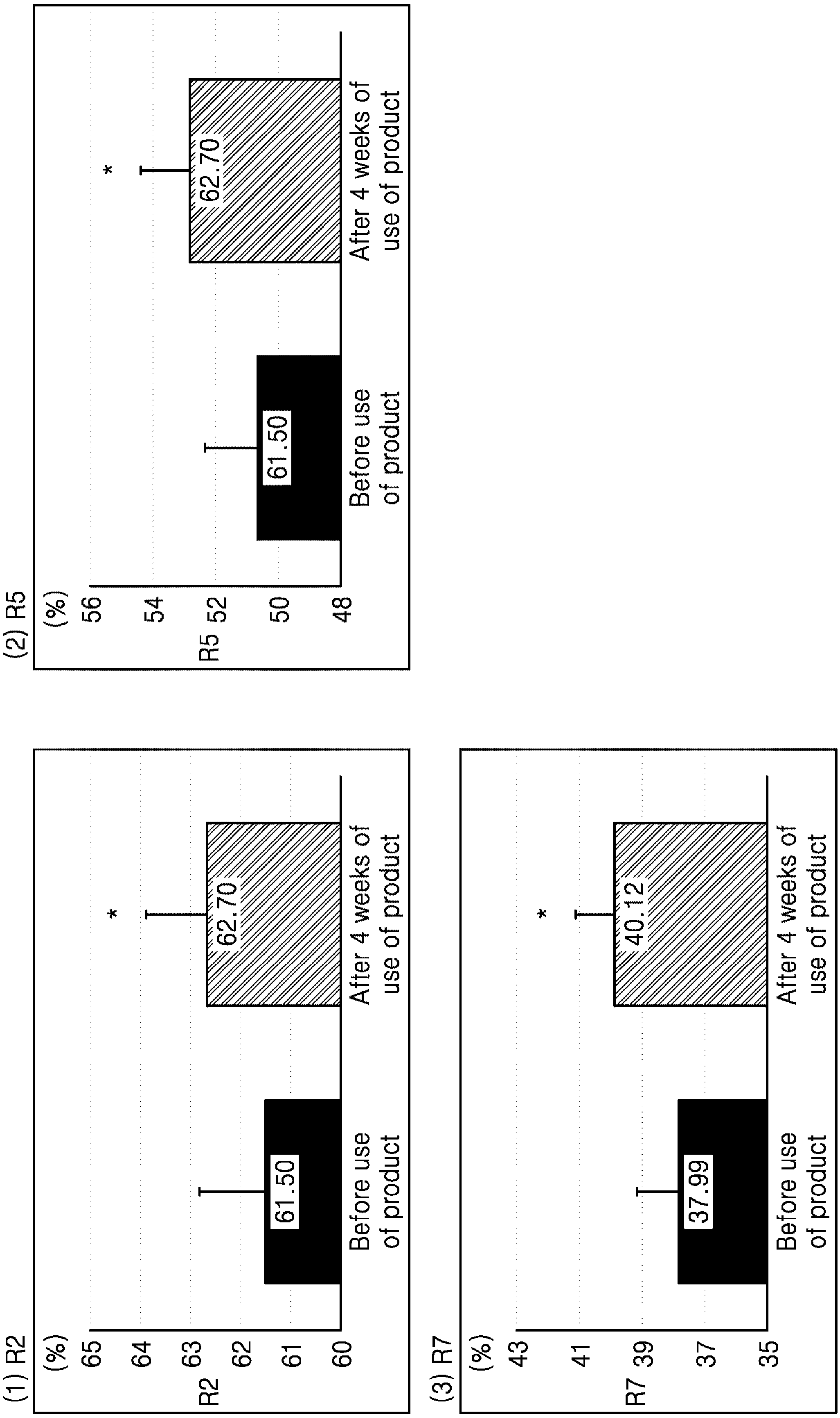
(1) R2
(%)
R2
65
64
63
62
61
60
61.50
62.70
*
Before use of product
After 4 weeks of use of product
(2) R5
(%)
R5
56
54
52
50
48
61.50
62.70
*
Before use of product
After 4 weeks of use of product
(3) R7
(%)
R7
43
41
39
37
35
37.99
40.12
*
Before use of product
After 4 weeks of use of product

FIG. 26

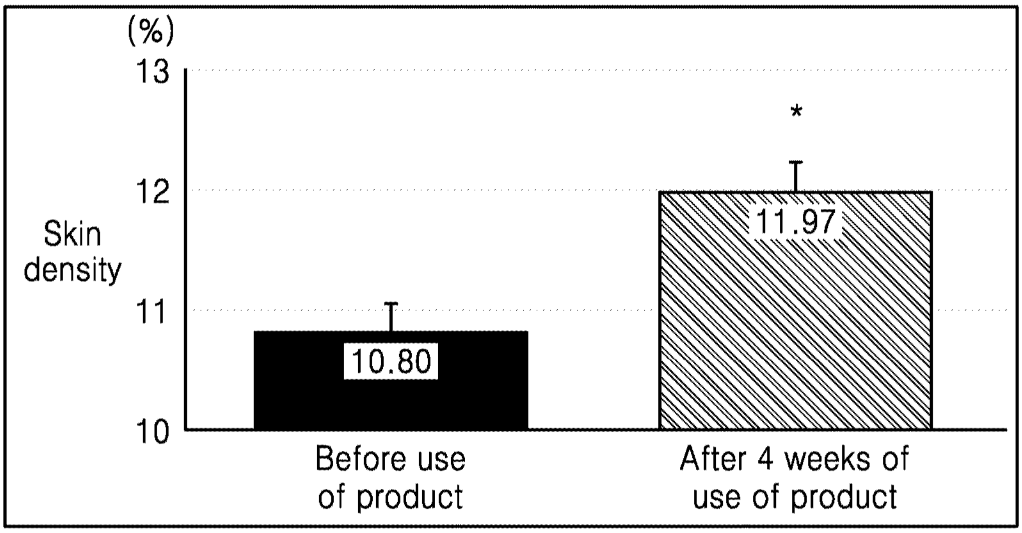
(%)
13
12
11
10
Skin density
*
11.97
10.80
Before use of product
After 4 weeks of use of product

FIG. 27

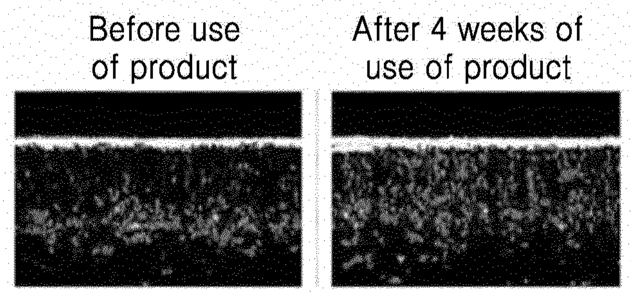

## INTERNATIONAL SEARCH REPORT

International application No. **PCT/KR2024/003544**

**A. CLASSIFICATION OF SUBJECT MATTER**

**C12N 1/20**(2006.01)i; **A61K 8/99**(2006.01)i; **A61Q 19/00**(2006.01)i

According to International Patent Classification (IPC) or to both national classification and IPC

**B. FIELDS SEARCHED**

Minimum documentation searched (classification system followed by classification symbols)

C12N 1/20(2006.01); A01N 63/02(2006.01); A61K 8/98(2006.01); A61K 8/99(2006.01); C07K 5/06(2006.01); C12N 5/02(2006.01); C12Q 1/02(2006.01); G01N 33/569(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 미생물 배양 배지 (microbial culture medium/solution), 인간 피부 유래 (human skin-derived products), 당류 (sugar), 글루코스 (glucose), 프룩토스 (fructose), 람노스 (rhamnose), 아미노산 (amino acids), 트레오닌 (threonine), 티로신 (tyrosine), 아르기닌 (arginine), 알라닌 (alanine), 프롤린 (proline), 히스티딘 (histidine), 이소류신 (isoleucine), 류신 (leucine), 페닐알라닌 (phenylalanine), 트립토판 (tryptophan), 발린 (valine), 글루탐산 (glutamic acid), 아스파라긴산 (aspartic acid), 세린 (serine), 글리신 (glycine), 스타필로코커스 에피데르미디스 (Staphylococcus epidermidis) KCCM12551P, 스트렙토코커스 써모필러스 (Streptococcus thermophilus) KCCM12004P, 큐티박테리움 아크네스 (Cutibacterium acnes) KCCM12680P, 코리네박테리움 아미콜라툼 (Corynebacterium amycolatum) KCCM12689P, 로티아 크리스티네 (Rothia kristinae) KCCM12685P

**C. DOCUMENTS CONSIDERED TO BE RELEVANT**

| Category* | Citation of document, with indication, where appropriate, of the relevant passages | Relevant to claim No. |
|---|---|---|
| A | WO 2013-070893 A1 (THE PROCTER & GAMBLE COMPANY) 16 May 2013 (2013-05-16)<br>See abstract; claims 1-10; and paragraphs [0044]-[0065] and [0071]. | 1-12 |
| A | KR 10-2014-0089805 A (M&C LIFE SCIENCE CO., LTD.) 16 July 2014 (2014-07-16)<br>See abstract; claims 1-5; and paragraphs [0032] and [0065]. | 1-12 |
| A | HOFREUTER, D. Defining the metabolic requirements for the growth and colonization capacity of Campylobacter jejuni. Frontiers in Cellular and Infection Microbiology. 2014, vol. 4, article no. 137, inner pp. 1-19.<br>See abstract; inner pages 4-6; figure 1; and table 1. | 1-12 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **21 June 2024** | **24 June 2024** |

Name and mailing address of the ISA/KR

**Korean Intellectual Property Office**
**Government Complex-Daejeon Building 4, 189 Cheongsa-ro, Seo-gu, Daejeon 35208**

Facsimile No. **+82-42-481-8578**

Authorized officer

Telephone No.

Form PCT/ISA/210 (second sheet) (July 2022)

INTERNATIONAL SEARCH REPORT

International application No.
PCT/KR2024/003544

C. DOCUMENTS CONSIDERED TO BE RELEVANT

| Category* | Citation of document, with indication, where appropriate, of the relevant passages | Relevant to claim No. |
|---|---|---|
| A | JP 2002-029929 A (HAKUTO CO., LTD.) 29 January 2002 (2002-01-29)<br>See abstract; and claims 1-6. | 1-12 |
| A | KR 10-2013-0106762 A (LIFE TECHNOLOGIES CORPORATION) 30 September 2013 (2013-09-30)<br>See abstract; claims 1-29; and paragraphs [0008], [0021] and [0087]. | 1-12 |
| A | KR 10-2018-0112255 A (KOREA RESEARCH INSTITUTE OF BIOSCIENCE AND BIOTECHNOLOGY) 12 October 2018 (2018-10-12)<br>See abstract; and claims 1-10. | 1-12 |
| PX | KR 10-2609654 B1 (COSMAX, INC.) 05 December 2023 (2023-12-05)<br>See claims 1-6, 8, 11 and 12.<br>(This document is a published earlier application that serves as a basis for claiming priority of the present international application.) | 1-12 |

Form PCT/ISA/210 (second sheet) (July 2022)

**INTERNATIONAL SEARCH REPORT**
**Information on patent family members**

International application No.
**PCT/KR2024/003544**

| Patent document cited in search report | | | Publication date (day/month/year) | Patent family member(s) | | | Publication date (day/month/year) |
|---|---|---|---|---|---|---|---|
| WO | 2013-070893 | A1 | 16 May 2013 | EP | 2776836 | A1 | 17 September 2014 |
| | | | | EP | 2776836 | B1 | 24 January 2018 |
| | | | | JP | 2014-533946 | A | 18 December 2014 |
| | | | | JP | 2014-533947 | A | 18 December 2014 |
| | | | | JP | 2014-534235 | A | 18 December 2014 |
| | | | | JP | 5922787 | B2 | 24 May 2016 |
| | | | | JP | 5969619 | B2 | 17 August 2016 |
| | | | | JP | 5969620 | B2 | 17 August 2016 |
| | | | | US | 2013-0115610 | A1 | 09 May 2013 |
| | | | | US | 2013-0115648 | A1 | 09 May 2013 |
| | | | | US | 2014-0072533 | A1 | 13 March 2014 |
| | | | | US | 8815538 | B2 | 26 August 2014 |
| | | | | US | 9271924 | B2 | 01 March 2016 |
| | | | | US | 9289376 | B2 | 22 March 2016 |
| | | | | WO | 2013-070891 | A1 | 16 May 2013 |
| | | | | WO | 2013-070894 | A1 | 16 May 2013 |
| KR | 10-2014-0089805 | A | 16 July 2014 | KR | 10-1426191 | B1 | 31 July 2014 |
| JP | 2002-029929 | A | 29 January 2002 | JP | 3803260 | B2 | 02 August 2006 |
| KR | 10-2013-0106762 | A | 30 September 2013 | AU | 2018-202612 | B2 | 29 October 2020 |
| | | | | AU | 2021-200418 | A1 | 25 February 2021 |
| | | | | AU | 2021-200418 | B2 | 25 May 2023 |
| | | | | CN | 102892878 | A | 23 January 2013 |
| | | | | CN | 106520658 | A | 22 March 2017 |
| | | | | DK | 3165600 | T3 | 05 December 2022 |
| | | | | DK | 3168295 | T3 | 21 November 2022 |
| | | | | EP | 3168295 | B1 | 02 November 2022 |
| | | | | EP | 4170016 | A1 | 26 April 2023 |
| | | | | JP | 2013-524817 | A | 20 June 2013 |
| | | | | JP | 6367293 | B2 | 01 August 2018 |
| | | | | JP | 7200180 | B2 | 06 January 2023 |
| | | | | KR | 10-1831549 | B1 | 22 February 2018 |
| | | | | KR | 10-2020-0051847 | A | 13 May 2020 |
| | | | | KR | 10-2109463 | B1 | 12 May 2020 |
| | | | | KR | 10-2264580 | B1 | 11 June 2021 |
| | | | | US | 11365389 | B2 | 21 June 2022 |
| | | | | US | 2011-0262965 | A1 | 27 October 2011 |
| | | | | US | 2017-058256 | A1 | 02 March 2017 |
| | | | | US | 2023-365919 | A1 | 16 November 2023 |
| | | | | WO | 2011-133902 | A2 | 27 October 2011 |
| | | | | WO | 2011-133902 | A3 | 19 April 2012 |
| KR | 10-2018-0112255 | A | 12 October 2018 | KR | 10-1920449 | B1 | 20 November 2018 |
| KR | 10-2609654 | B1 | 05 December 2023 | | None | | |

Form PCT/ISA/210 (patent family annex) (July 2022)